# EUROPÄISCHE PATENTSCHRIFT

(11) **EP 3 055 663 B1**
(45) Veröffentlichungstag und Bekanntmachung des Hinweises auf die Patenterteilung: **01.12.2021**
(21) Anmeldenummer: 14781176.4
(22) Anmeldetag: 06.10.2014
(51) Int. Cl.: G01N 1/14, B65B 57/14, B65B 3/00, C12M 1/26, G01N 1/20

(54) **ENTNAHMEVORRICHTUNG, VERFAHREN UND VERWENDUNG ZUM GEWINNEN EINER PROBE EINES ZU ENTNEHMENDEN MEDIUMS**
EXTRACTION DEVICE, METHOD AND USE FOR OBTAINING A SAMPLE OF A MEDIUM TO BE EXTRACTED
DISPOSITIF DE PRÉLÈVEMENT, PROCÉDÉ ET UTILISATION POUR OBTENIR UN ÉCHANTILLON D'UN MILIEU À PRÉLEVER

(30) Priorität: 08.10.2013 DE 102013220290
(43) Veröffentlichungstag der Anmeldung: 17.08.2016
(73) Patentinhaber: Sartorius Stedim Biotech GmbH, 37079 Göttingen (DE)
(72) Erfinder: KÜNNECKE, Wolfgang, 38102 Braunschweig (DE); PREDIGER, Andreas, 37073 Göttingen (DE)
(74) Vertreter: Vigand, Philippe
(86) Internationale Anmeldenummer: PCT/EP2014/071345
(87) Internationale Veröffentlichungsnummer: WO 2015/052134

(56) Entgegenhaltungen:
- DE-A1- 10 336 539
- US-A- 3 656 349
- US-A- 5 101 670
- US-A- 5 172 332
- US-A1- 2002 170 364
- US-A1- 2009 305 407
- US-A1- 2010 236 340
- US-A1- 2013 240 082
- US-B1- 7 874 221
- Anonymous: "Peristaltic pump - Wikipedia", , 23 May 2020 (2020-05-23), XP055706826, Retrieved from the Internet: URL:https://en.wikipedia.org/wiki/Peristal tic_pump [retrieved on 2020-06-19]
- Physik Instrumente USA - Precision Motion Control: "Piezo Pump, Ultrasonic for Medical Engineering, by www.pi-medical.net", YouTube, 14 December 2010 (2010-12-14), pages 1-1, XP054980602, Retrieved from the Internet: URL:https://www.youtube.com/watch?v=CSqUSt RTBA0 [retrieved on 2020-06-19]

## Beschreibung

Die Erfindung betrifft eine Entnahmevorrichtung zum Gewinnen einer Probe eines zu entnehmenden Mediums aus einem Prozessstrom, ein Verfahren zum Gewinnen einer Probe eines zu entnehmenden Mediums aus einem Prozessstrom, eine Schlauchsystem-Anordnung sowie die Verwendung einer Entnahmevorrichtung.

Entnahmevorrichtungen der eingangs genannten Art weisen in der Regel eine Entnahmeleitung, meist in Form eines Entnahmeschlauchs, auf, sowie ein Quetschventil zum Verschließen der Entnahmeleitung. Die Entnahmevorrichtungen sind meist Teil eines Schlauchsystems, das eine Mehrzahl von Behältern miteinander verbindet und auch unter dem Namen "Manifold" bekannt ist. Solche Schlauchsysteme bestehen aus einer Hauptleitung, von der mehrere Entnahmeleitungen abzweigen, die dann jeweils mit einem Behälter verbunden sind. Der Einlass der Hauptleitung ist beispielsweise mit einem Vorratsbehälter, der Teil eines bspw. zu analysierenden oder zu überwachenden Prozesses ist, verbunden. Über den Einlass gelangt zu entnehmendes Medium in die Hauptleitung, von welcher aus dieses dann in die unterschiedlichen Entnahmeleitungen strömt.

Insbesondere bei Herstellungsprozessen von biologisch erzeugten Pharmawirkstoffen und dergleichen werden solche Schlauchsysteme als Einwegsysteme verwendet, die als vorkonfigurierte, sterile Produkte angeboten werden. Durch die Nutzung von Einwegsystemen entfallen zeitaufwändige und kostenintensive Reinigungs- und Validierungsschritte. Auch in anderen Bereichen werden Einwegsysteme, auch "Single-use"-Systeme genannt, zunehmend verwendet.

Zur automatisierten Entnahme von zu entnehmendem Medium aus einem Prozessstrom werden die Schlauchsysteme in Verbindung mit geeigneten Ventilanordnungen genutzt. Als Ventile werden hierzu in der Regel Quetschventile eingesetzt, die jeweils an einer Entnahmeleitung angeordnet sind und so gesteuert werden, dass nur dasjenige Quetschventil geöffnet wird, über dessen zugehörige Entnahmeleitung zu entnehmendes Fluid in den entsprechenden Behälter geleitet werden soll. Eine derartige Vorrichtung ist beispielsweise aus EP 1 525 138 B1 bekannt. Als Behälter dienen dabei flexible Kunststoffbeutel, die wiederum mit einem flexiblen Verbindungsschlauch an eine mit zu entnehmendem Medium beströmte Hauptleitung angeschlossen werden. Jedem Behälter ist ein eigener Verbindungsschlauch zugeordnet. Das Schlauchsystem wird in eine Vorrichtung eingelegt, die mittels Quetschventilen den Eintritt der Flüssigkeit in den jeweiligen Behälter steuert.

US 2010 0236340 offenbart ein geschlossenes Probennahmesystem, das mittels einer Pumpe Flüssigkeit in eine Hauptleitung transportiert und diese mittels des Schlauchsystems und Quetschventilen in verschiedene Beutel aufteilt. Zusätzlich können mittels Quetschventilen gefilterte Entlüftungsleitungen zugeschaltet werden, um die Hauptleitung und die Entnahmeleitungen zu entlüften, ohne das System zu öffnen.

Ein im Unterschied dazu offenes System ist in DE 10 2010 060 469 offenbart, bei dem ein gasdurchlässiger flüssigkeitsdichter Sterilfilter verwendet wird, durch den Luft zum Entlüften der Hauptleitung in das System eingeführt werden kann.

DE 10 2011 001 584 offenbart ein weiteres Schlauchsystem mit einer Hauptleitung, mehreren Entnahmeleitungen, die mit flexiblen Beuteln verbunden sind, sowie einem Ventilträger mit einer Mehrzahl von Ventilen, umfassend eine der Mehrzahl der Zielbehälter entsprechenden Mehrzahl von Zielbehälterventilen, mittels denen jeweils ein Volumenfluss durch eine der Entnahmeleitungen steuerbar ist. Ferner lässt sich die Hauptleitung mittels steril gefiltertem Gas und einer Gasdruckquelle vollständig entleeren.

US 2009/0305407 A1 offenbart eine Entnahmeanordnung, in der eine Hauptleitung eines Schlauchsystems, die eine biologische Flüssigkeit aufnimmt, selektiv durch eine Vielzahl von Sammelkammern beprobt wird. Die selektive Probenahme erfolgt durch selektiven Zugriff auf die Hauptleitung und selektives Einschalten einer Probenahmepumpe durch einen Mikroprozessor. Insbesondere offenbart dieses Dokument eine Entnahmevorrichtung zum Gewinnen einer Probe eines zu entnehmenden Mediums aus einem Prozessstrom, umfassend: eine Entnahmeleitung, die mit einer Hauptleitung eines Schlauchsystems verbindbar oder lösbar verbunden ist, zum Leiten von zu entnehmendem Medium aus der Hauptleitung in einen Behälter; und eine Pumpe, zum Pumpen von zu entnehmendem Medium aus der Hauptleitung in den Behälter, wobei die Pumpe im Ruhezustand einen mediendichten Verschluss der Entnahmeleitung bildet.

US 5,101,670 A offenbart eine Entnahmevorrichtung, die einen spritzenartigen Probenbehälter mit einem Einlass/Auslass und einen darin befindlichen Kolben umfasst. Der Einlass des Probenbehälters ist mit einem Fluidstrom verbindbar, um Proben in den Behälter zu ziehen. Der Probenbehälter ist von dem Fluidstrom abkoppelbar, um ein Überführen des Probenbehälters zu einem Probenanalysegerät zu ermöglichen.

US 7,874,221 B1 offenbart ein Probenpumpensystem zur Dosierung einer Probenmenge, die aus einem unter Druck stehenden Fluidprozess entnommen wird. Das Probenpumpensystem ist zum direkten oder indirekten Eintauchen in den unter Druck stehenden Fluidstrom ausgelegt, so dass die Probenahme bei dem vorherrschenden Druck und Temperatur des Fluidstroms erfolgt.

Nachteilig bei der Verwendung von Quetschventilen ist allerdings, dass diese zur Entnahme des Schlauchsystems wieder geöffnet werden müssen. Um hier ein Verunreinigen der Probe zu verhindern, ist es erforderlich, die einzelnen Entnahmeleitungen vor Öffnen der Quetschventile zu verschließen, etwa mittels Klemmen oder mittels Verschweißen der Entnahmeleitung. Vergisst ein Anwender, die separaten Klemmen an den Quetschventilen zu setzen, oder kommt es zu einer Fehlanwendung, können sich die Medien in dem Schlauchsystem unkontrolliert bewegen, und die Qualität der Proben ist nicht mehr sichergestellt. Es kann also zu einem sogenannten unsicheren Zustand des Systems kommen, bei dem sich die entnommenen Proben vermischen. Ferner kann die Verwendung von Quetschventilen dazu führen, dass die Entnahmeleitung beschädigt wird oder sich nach Entfernen des Quetschventils nicht mehr von selbst öffnet.

Ein weiterer Nachteil der bisher bekannten Schlauchsysteme ist, dass in der Abzweigung von der Hauptleitung zur Entnahmeleitung ein Totvolumen entsteht. Das Volumen dieses Bereichs wird durch die geometrischen Dimensionen zwischen dem Übergang von Hauptleitung und Entnahmeleitung und der Stelle der Abklemmung der Entnahmeleitung im Quetschventil bestimmt. Da das Quetschventil nicht unmittelbar in der Übergangsstelle zwischen Entnahmeleitung und Hauptleitung liegen kann, entsteht bei der Verwendung von Quetschventilen im Schlauchsystem immer ein Totvolumen in diesem Bereich. Bei Verwendung von mehreren Entnahmeleitungen an einer Hauptleitung und Entnahmen in zeitlicher Abfolge können Restmengen in den Totvolumina der einzelnen Entnahmeleitungen dazu führen, dass die nachfolgend entnommene Probe mit Medium von einem früheren Zeitpunkt verunreinigt ist.

Aufgabe der vorliegenden Erfindung ist es daher, eine gegenüber den vorbekannten Vorrichtungen und Schlauchsystemen verbesserte Entnahmevorrichtung anzugeben, bei der insbesondere nicht die Gefahr besteht, dass die Proben nach Abschluss der Entnahme vermischt werden und bei der vorzugsweise Totvolumina verhindert oder minimiert werden.

Erfindungsgemäß wird eine Entnahmevorrichtung zum Gewinnen einer Probe eines zu entnehmenden Mediums aus einem Prozessstrom angegeben, umfassend eine Entnahmeleitung, die mit einer Hauptleitung eines Schlauchsystems verbindbar oder lösbar verbunden ist, zum Leiten von zu entnehmendem Medium aus der Hauptleitung in einen Behälter; und eine in der Entnahmeleitung angeordnete Pumpe, zum Pumpen von zu entnehmendem Medium aus der Hauptleitung in den Behälter, wobei die Pumpe im Ruhezustand einen mediendichten Verschluss der Entnahmeleitung bildet, wobei die Entnahmeleitung aus einem ersten und einem zweiten Abschnitt besteht, wobei der erste Abschnitt die Hauptleitung medienleitend mit einem Pumpeneinlass verbindet und der zweite Abschnitt einen Pumpenauslass medienleitend mit dem Behälter verbindet, sodass ein Strömungspfad von der Hauptleitung in den Behälter durch die Pumpe verläuft. Damit wird erfindungsgemäß anstelle eines Quetschventils zum Verschließen der Entnahmeleitung eine Pumpe eingesetzt. Die Pumpe bildet im Ruhezustand einen mediendichtenden Verschluss der Entnahmeleitung. Vorzugsweise ist die Pumpe in miniaturisierter Form ausgebildet. Dadurch ist die Entnahmevorrichtung raumsparend ausgebildet.

Das zu entnehmende Medium kann etwa ein zu analysierendes Medium sein oder als Teilmenge oder Aliquot zu Aufbewahrungszwecken oder als Produkt (Endprodukt oder Zwischenprodukt) entnommen werden.

Unter Entnahmeleitung wird vorliegend die Verbindungsleitung zwischen einer Hauptleitung und einem Behälter zum Aufnehmen der Probe verstanden. Die Entnahmeleitung kann einstückig mit der Hauptleitung und/oder mit dem Behälter verbunden sein. Alternativ kann die Entnahmeleitung an einer dafür vorgesehenen Verbindungsstelle mit der Hauptleitung verbindbar bzw. verbunden sein und/oder mit einer dafür vorgesehenen Verbindungsstelle an dem Behälter verbindbar bzw. verbunden sein. Weiter alternativ kann die Entnahmeleitung mittels kraft- oder formschlüssiger Verbindung mit einer Öffnung in der Hauptleitung verbindbar bzw. verbunden sein.

Erfindungsgemäß ist dabei vorgesehen, dass die Pumpe in der Entnahmeleitung angeordnet ist. Die Pumpe bildet demnach einen Teil der Entnahmeleitung, sodass ein Strömungspfad von einer Hauptleitung in einen Behälter durch die Pumpe verläuft und zu entnehmendes Medium von dieser Pumpe gefördert wird.

Die Entnahmeleitung ist beispielsweise und bevorzugt als Schlauch ausgebildet. Ferner ist bevorzugt, dass der Pumpeneinlass unmittelbar mit einer Hauptleitung verbindbar bzw. verbunden ist und der Pumpenauslass mit einer Entnahmeleitung verbindbar bzw. verbunden ist, welche wiederum mit einem Behälter verbunden ist. Ferner ist bevorzugt, dass der Pumpenauslass direkt mit dem Behälter verbunden ist. Die Verbindungen können mittels bekannter Verbindungstechniken realisiert werden. Beispielsweise aber nicht abschließend mittels Ultraschall, thermischem oder Laserschweißen, Kleben oder mechanischen Verbindungsmitteln, wie etwa Schnallen, Klemmen, Bindern oder Klips. Die Verbindung zwischen der Entnahmeleitung mit einer Hauptleitung wird bevorzugt über konventionelle (Schlauch-)Verbinder hergestellt. Diese können lösbar oder unlösbar ausgebildet sein. Ferner wird bevorzugt steriles Verschweißen zum Verbinden der Leitungen eingesetzt.

Die Hauptleitung kann an eine Prozessanlage (etwa Behälter, Reaktor, Leitung), in der ein Prozess stattfindet, angeschlossen werden oder sein. Die Hauptleitung kann auch selbst einen Teil der Prozessanlage bilden, beispielsweise bei einer Abfüllanlage. Das heißt, dass die Hauptleitung auch ein Reaktor, insbesondere ein Bioreaktor sein kann, wobei ein solcher Bioreaktor bevorzugt als fester single-use Bioreaktor, flexibler single-use bag oder als klassischer Bioreaktor (z.B. aus aus Edelstahl) ausgeformt ist. Zum Anschluss der Hauptleitung an den Prozess werden bevorzugt aseptische Verbinder verwendet. Ferner wird bevorzugt steriles Verschweißen zum Verbinden der Leitungen eingesetzt.

Geeignete im Rahmen der vorliegenden Erfindung zu verwendende Behälter umfassen insbesondere flexible Kunststoffbeutel, Gefäße und Flaschen aus Kunststoff oder Glas, sowie Spritzen oder andere bekannte Probengefäße.

Als Pumpen können beispielsweise Pumpen vom Typ Rotor/Stator, Kolbenpumpen oder Verdrängerpumpen, wie etwa Zahnradpumpen, eingesetzt werden. Vorzugsweise weist eine derartige Pumpe eine Dimension entsprechend ihrer Größenausdehnung auf, die nicht größer ist als 10 cm, 5 cm, 3 cm oder 2 cm, insbesondere 1,5 cm, besonders bevorzugt 1 cm. Eine Pumpengröße in einem Bereich zwischen 3 cm und 10 cm ist bevorzugt bei einem zu entnehmenden Medium, welches als Aliquot oder Produkt verwendet wird; eine Pumpengröße in einem Bereich zwischen 3 cm und 1 cm oder kleiner ist bevorzugt bei einem zu entnehmenden Medium, welches als analytische Probe dient.

Indem eine Pumpe in der Entnahmeleitung vorgesehen ist, ist die Entnahmeleitung auch nach Entfernen eines Schlauchsystems, in welchem die Entnahmevorrichtung verwendet wird, mediendicht, und ein unsicherer Zustand tritt nicht ein. Ferner hat die Pumpe den Vorteil, dass ein Medienstrom von der Hauptleitung in einen Behälter nicht abhängig von der Druckdifferenz zwischen Hauptleitung und Behälter ist, sondern mittels der Pumpe bewirkt wird. Zusätzlich kann die Pumpe näher an einer Übergangsstelle zwischen einer Hauptleitung und einer Entnahmeleitung angeordnet sein, wodurch ein Totraum verkleinert wird.

Gemäß einer ersten bevorzugten Ausführungsform der Erfindung ist das Schlauchsystem ein "Manifold"-Schlauchsystem zur Entnahme und/oder Aufbewahrung von Proben und/oder Aliquoten aus einem biotechnologischen, pharmazeutischen, medizinischen oder lebensmitteltechnischen Herstellungsprozess.

Ferner ist bevorzugt, dass die Pumpe einen Pumpeneinlass und einen Pumpenauslass aufweist, wobei der Pumpeneinlass englumig ausgebildet ist. Englumig bedeutet vorliegend, dass der Pumpeneinlass relativ zu dem Pumpenauslass, der Entnahmeleitung und/oder der Hauptleitung einen kleine(re)n Durchmesser aufweist. Der Durchmesser ist vorzugsweise derart klein, dass zu entnehmendes Medium aus der Hauptleitung nicht bzw. im Wesentlichen nicht unter Gravitationseinwirkung alleine in die Entnahmeleitung strömt. Hierdurch wird das Totvolumen in der Entnahmeleitung wesentlich verringert, und ein Vermischen von unterschiedlichen Proben wird vermieden. Zu entnehmendes Medium strömt nur unter Einwirkung der Pumpe in die Entnahmeleitung und somit das Totvolumen.

Gemäß einer weiteren bevorzugten Ausführungsform umfasst die Entnahmevorrichtung ferner eine Spüleinrichtung, vorzugsweise eine mit der Entnahmeleitung verbundene Spülmediumzuführleitung, zum Zuführen von Spülmedium in die Entnahmeleitung. Als Spülmedium kann etwa Luft, sterilisierte Luft oder ein anderes steriles Gas verwendet werden. Auch können hierzu Flüssigkeiten, insbesondere sterile Flüssigkeiten, verwendet werden. Vorzugsweise ist eine Spülmediumzuführleitung vorgesehen, die an der Pumpe oder zwischen Pumpe und einem Beutel in die Entnahmeleitung mündet. So ist das Spülmedium mittels der Pumpe durch die Entnahmeleitung in Richtung der Hauptleitung pumpbar, um so einen Abschnitt der Entnahmeleitung zwischen einem Pumpeneinlass und der Hauptleitung und/oder die Hauptleitung freizuspülen. Gemäß einer solchen Ausführungsform ist vorzugsweise ferner ein Rückschlagventil in einer Spülmediumzuführleitung vorgesehen, so dass ein Einströmen von zu entnehmendem Medium in die Spülmediumzuführleitung verhindert wird. Vorzugsweise wird hierzu ein doppeltes Rückschlagventil, ein sogenanntes Dual-Rückschlagventil oder "dual-check-valve" (DCV) benutzt.

Vorzugsweise ist ferner ein steriler Filter an der Spüleinrichtung vorgesehen. Dadurch ist es möglich, Umgebungsluft über den sterilen Filter anzusaugen und so sterile Luft als Spülmedium in die Entnahmevorrichtung einzuführen. Gemäß einer solchen Ausführungsform verhindert ein Rückschlagventil ferner den Kontakt von zu entnehmendem Medium mit dem sterilen Filter, so dass dieser nicht kontaminiert wird.

Gemäß einer bevorzugten Weiterbildung ist die Entnahmeleitung mit einem Ende fest mit der Hauptleitung verbunden und weist an diesem, mit der Hauptleitung verbundenen Ende ein Rückhaltemittel auf. Das Rückhaltemittel verhindert einen selbsttätigen Medieneintritt in die Entnahmeleitung. Vorzugsweise ist das Rückhaltemittel als Verschluss, insbesondere als Kappe ausgebildet, die mittels eines Überdrucks öffenbar ist, welcher mittels der Pumpe auf die Kappe aufbringbar ist. Beispielsweise ist die Kappe aus einem elastischen Kunststoff, beispielsweise Silikon, gebildet, sodass die Kappe beim Pumpen aufgebläht wird und schließlich platzt.

Der erfindungsgemäße Aspekt des Rückhaltemittels stellt auch unabhängig von der Verwendung einer Pumpe in der Entnahmeleitung einen eigenständigen Aspekt dar. Folglich wird die eingangs genannte Aufgabe gemäß einem Aspekt der Erfindung gelöst durch eine Entnahmevorrichtung zum Gewinnen einer Probe eines zu entnehmenden Mediums aus einem Prozessstrom, umfassend: eine Entnahmeleitung, die mit einer Hauptleitung eines Schlauchsystems verbindbar oder verbunden ist, zum Leiten von zu entnehmendem Medium aus der Hauptleitung in einen Behälter; und ein an dem mit der Hauptleitung verbindbaren oder verbundenen Ende angeordnetem Rückhaltemittel. Bei einer derartigen Ausgestaltung können auch Quetschventile eingesetzt werden. Das Totvolumen in der Entnahmeleitung ist durch das Rückhaltemittel gegen Eintritt von Medium abgeschlossen. Vorzugsweise wird dieser Aspekt mit weiteren bevorzugten Ausgestaltungen wie sie vorstehend und nachstehen beschrieben sind kombiniert.

In einer weiteren bevorzugten Alternative umfasst das Rückhaltemittel eine hydrophobe Beschichtung auf einer inneren Oberfläche der Entnahmeleitung. Alternativ ist die Entnahmeleitung wenigstens abschnittsweise aus einem hydrophoben Material gebildet. Vorzugsweise ist die Beschichtung in der Entnahmeleitung in einem Bereich zwischen der Hauptleitung und der Pumpe, vorzugsweise unmittelbar an der Hauptleitung ausgebildet.

Gemäß einer bevorzugten Weiterbildung ist in der Entnahmeleitung, vorzugsweise in einem Übergangsbereich zwischen Hauptleitung und Entnahmeleitung eine hydrophobe Membran angeordnet. Die Membran ist vorzugsweise erst bei einer vorbestimmten Druckdifferenz mediendurchlässig. Eine derartige Membran ist bevorzugt als Filter oder Netz ausgebildet. So lassen sich mittels den Porengrößen der Filter oder Netze auf einfache Art und Weise vorbestimmte Druckdifferenzen (Durchtrittsdrücke) einstellten.

Als hydrophobe Materialien können etwa Polytetrafluorethylen oder Polypropylen eingesetzt werden.

Durch ein Rückhaltemittel wird verhindert, dass zu entnehmendes Medium in ein Totvolumen der Entnahmeleitung eindringen kann und so nachfolgende Proben verunreinigt. Das Totvolumen wird erst unmittelbar vor Entnahme der der entsprechenden Entnahmeleitung zugeordneten Probe geöffnet bzw. zugänglich. Dadurch wird die Qualität der Proben erhöht.

In einer weiteren bevorzugten Ausgestaltung der Erfindung ist an einem Ende der Entnahmeleitung eine Hohlnadel zum Durchstechen einer Wandung einer Hauptleitung angeordnet. Gemäß dieser Ausführungsform ist die Entnahmeleitung nicht fest oder vorkonfektioniert mit der Hauptleitung verbunden. Vielmehr bietet diese Ausführungsform dem Anwender und auch Herstellern eines solchen Manifolds die Möglichkeit, die Entnahmeleitung entsprechend seines jeweiligen Anwendungsfalls mit der Hauptleitung zu verbinden, indem der Anwender mit der Hohlnadel die Wandung der Hauptleitung durchstößt und so eine medienleitende Verbindung zwischen Hauptleitung und Entnahmeleitung herstellt. Die Hohlnadel bildet gemäß dieser Ausführungsform einen Abschnitt der Entnahmeleitung. Diese Ausführungsform der Erfindung hat ferner den Vorteil, dass die Entnahmevorrichtung ein preisgünstiges und vielseitig konfektionierbares Element darstellt, welches dem Anwender ermöglicht, ein Schlauchsystem auf einfache Art und Weise mit einer Anzahl an Entnahmeleitungen zu bilden, die seinem Anwendungsfall entsprechen. Bevorzugt weist die Hohlnadel eine derartige axiale Länge auf, dass sie eine Wandung einer Hauptleitung vollständig durchdringen kann, jedoch nur ein kurzes Stück in das Innere der Hauptleitung ragt. Dadurch ist das Totvolumen, welches sich in der Hohlnadel bildet, weiter reduziert. Die Hohlnadel ist bevorzugt aus Kunststoff und/oder einem anderen inerten Material wie etwa Edelstahl oder Carbonwerkstoff gebildet. Bevorzugt ist sie mittels Spritzgießen an die Pumpe, insbesondere einen Einlass eines Pumpenkörpers angespritzt. Bevorzugt ist die Hohlnadel mit einem Pumpenkörper einteilig insbesondere mittels Spritzgießen hergestellt.

Ferner ist gemäß dieser Ausführungsform vorzugsweise ein Stützelement vorgesehen, welches benachbart zu der Hohlnadel angeordnet ist und dazu ausgebildet ist, nach einem Durchstechen einer Wandung einer Hauptleitung mit dieser in Kontakt zu kommen, um die Entnahmeleitung gegen die Hauptleitung abzustützen und/oder abzudichten. Besonders bevorzugt sind an dem Stützelement Klebemittel vorgesehen, mittels derer das Stützelement gegen eine äußere Wandung der Hauptleitung klebbar ist. So wird eine dauerhaftere und stabilere Verbindung zwischen der Entnahmeleitung und der Hauptleitung gebildet. Dies führt zu einer erhöhten Sicherheit und einer verbesserten Probenqualität. Die Stützvorrichtung kann auch dergestalt ausgeführt sein, dass die Entnahmevorrichtung am Schlauch der Hauptleitung mittels Kabelbinder befestigt werden kann.

In einer weiteren Ausführungsform weist die Entnahmevorrichtung einen Schlauchverbinder auf. Der Schlauchverbinder ist vorzugsweise als T-Stück ausgebildet. Bevorzugt ist der Schlauchverbinder mit dem Ende der Entnahmeleitung oder direkt mit dem Pumpeneinlass verbunden, vorzugsweise vorkonfektioniert. So können an zwei freien Enden des Schlauchverbinders Leitungsstücke angeordnet werden, um so eine Hauptleitung zu bilden.

Gemäß einer weiteren bevorzugten Ausführungsform weist die Entnahmevorrichtung ein Selektorventil auf, welches einlassseitig mit der Hauptleitung und auslassseitig mit zwei oder mehr Behältern verbunden ist, wobei das Selektorventil schaltbar ist, zum wahlweisen Verbinden der Entnahmeleitung mit einem Behälter der zwei oder mehr Behälter. Vorzugsweise ist das Selektorventil in der Entnahmeleitung angeordnet. Vorzugsweise ist das Selektorventil jeweils mittels der Entnahmeleitung mit der Hauptleitung und den Behältern verbunden. Die Entnahmeleitung hat demnach stromabwärts des Selektorventils zwei oder mehr Zweige, entsprechend der Anzahl der Behälter. Mittels des Selektorventils kann dann zwischen verschiedenen Zweigen der Entnahmeleitung geschaltet werden, so dass zu entnehmendes Medium aus der Hauptleitung in wahlweise einen der zwei oder mehr Behälter strömen kann. Vorzugsweise sind drei oder mehr Behälter, vier oder mehr Behälter, besonders bevorzugt fünf oder mehr Behälter an dem Selektorventil vorgesehen. Das Selektorventil kann manuell oder elektrisch betätigt werden.

In einer bevorzugten Weiterbildung ist vorgesehen, dass die Pumpe der Entnahmeleitung zwischen dem Selektorventil und der Hauptleitung angeordnet ist. Dadurch wird mit derselben Pumpe Medium von der Hauptleitung in jeden der zwei oder mehr Behälter, entsprechend der Stellung des Selektorventils, gepumpt.

In einer Alternative weist die Entnahmevorrichtung zwei oder mehr Pumpen auf, wobei jeweils eine Pumpe der zwei oder mehr Pumpen zwischen dem Selektorventil und einem der zwei oder mehr Behälter angeordnet ist. Demnach ist in jedem Zweig der Entnahmeleitung zwischen dem Selektorventil und den jeweiligen Behältern eine eigene Pumpe vorgesehen. Es sind demnach so viele Pumpen vorgesehen wie Behälter an dem Selektorventil angeschlossen sind. Hierdurch ist jedem Behälter eine eigene Pumpe zugeordnet, mittels der, bei entsprechender Schaltung des Selektorventils, Medium aus der Hauptleitung durch die Entnahmeleitung in den Behälter pumpbar ist. Gemäß einer solchen Ausführungsform ist vorgesehen, dass die Pumpe im Ruhezustand einen mediendichten Verschluss bildet. Dadurch ist es möglich, nach Abschluss einer Entnahme einen entsprechenden Behälter samt dem ihm zugeordneten Zweig der Entnahmeleitung, in dem die Pumpe angeordnet ist, von dem Selektorventil zu entfernen, ohne dass die entnommene Probe aus dem Behälter entweicht.

Gemäß einer weiteren bevorzugten Ausführungsform ist die Spüleinrichtung mit dem Selektorventil verbunden ist. Das Selektorventil weist mehrere Anschlüsse auf, die sowohl als Einlass als auch als Auslass dienen können. Beispielsweise ist ein erster Anschluss des Selektorventils über einen Abschnitt der Entnahmeleitung mit der Hauptleitung verbunden und über drei Zweige der Entnahmeleitung sind drei Behälter mit zweiten, dritten und vierten Anschlüssen des Selektorventils verbunden. An einen fünften Anschluss ist die Spüleinrichtung mittels der Spülmediumzuführleitung angeschlossen. Dadurch ist es möglich vor und/oder nach Entnahme eines Medium aus der Hauptleitung in einen der Behälter den Abschnitt der Entnahmeleitung zwischen dem Selektorventil und der Hauptleitung zu spülen. Dazu ist das Selektorventil entsprechend zu schalten und die Pumpe so zu betätigen, dass ein Spülmedium durch die Spülmediumzuführleitung, das Selektorventil und den Abschnitt der Entnahmeleitung zwischen Selektorventil und Hauptleitung gepumpt wird. Es hat sich herausgestellt, dass es in der Regel ausreichend ist den Abschnitt der Entnahmeleitung zwischen Selektorventil und Hauptleitung zu spülen. Gemäß dieser Ausführungsform ist nur eine Spüleinrichtung für eine Mehrzahl an Behältern erforderlich, wodurch Herstellungskosten und die Größe der Entnahmevorrichtung reduziert sind.

Vorzugsweise ist in einer Alternative oder zusätzlich ferner vorgesehen, dass die Spülmediumzuführleitung der Spüleinrichtung, zum Zuführen von Spülmedium in die Entnahmeleitung, zwischen dem Selektorventil und wenigstens einem Behälter mit der Entnahmeleitung verbunden ist. Vorzugsweise sind mehrere Spüleinrichtungen vorgesehen, besonders bevorzugt ist je Behälter eine Spüleinrichtung vorgesehen, sodass mit jedem Zweig der Entnahmeleitung zwischen dem Selektorventil und dem jeweiligen Behälter eine Spülmediumzuführleitung verbunden ist. Dadurch weist jeder Behälter und so jeder Zweig der Entnahmeleitung zwischen dem Selektorventil und dem jeweiligen Behälter eine eigene separate Spüleinrichtung auf. Dies ist besonders bevorzugt, wenn nur eine Pumpe vorgesehen ist, die zwischen der Hauptleitung und dem Selektorventil angeordnet ist. In einem solchen Fall kann die Pumpe mit der jeweiligen Spüleinrichtung des zu befüllenden Behälters rückgespült werden, um Verunreinigungen zu vermeiden. Die Pumpe dient in einem solchen Fall vorzugsweise dazu, das Spülmedium anzusaugen und entgegen der Entnahmevorrichtung durch die Entnahmeleitung zu pumpen.

Weiterhin ist bevorzugt, dass eine oder mehrere weitere Spüleinrichtungen vorgesehen sind, wobei jedem Behälter der zwei oder mehr Behälter eine Spüleinrichtung zugeordnet ist, derart dass die Spülmediumzuführleitung der dem jeweiligen Behälter zugeordneten Spüleinrichtung zum Zuführen von Spülmedium in die Entnahmeleitung, zwischen dem Selektorventil und dem jeweiligen Behälter, mit der Entnahmeleitung verbunden ist. Gemäß dieser Ausführungsform weist jeder Behälter eine separate Spüleinrichtung auf. Die Spülmediumzuführleitung mündet dann in den Zweig des Entnahmeleitung, die das Selektorventil mit dem entsprechenden Behälter verbindet. Dadurch ist jeder Zweig vor einer Probennahme separat rückspülbar.

Gemäß einer weiteren bevorzugten Ausführungsform ist eine Injektoreinrichtung zum Injizieren eines Reagenz in die Entnahmeleitung vorgesehen. Bei manchen Proben ist es erforderlich, diese vor der weiteren Verarbeitung beziehungsweise Vermessung mit geeigneten Sensoren mit einem oder mehreren Fluiden oder Reagenzien wie z.B. Enzymlösung, Färbelösung, Detergenz, Säure, Base oder Verdünnungsfluid wie Wasser und/oder Pufferlösung zu vermischen. Daher ist es bevorzugt, an der Entnahmevorrichtung eine geeignete Injektoreinrichtung vorzusehen, mittels der entsprechenden Fluide direkt in die Entnahmeleitung und somit in den Behälter injizierbar sind

Die Injektoreinrichtung weist vorzugsweise ein Injektorventil auf, welches in der Entnahmeleitung zwischen der Pumpe und dem Behälter angeordnet ist. Alternativ ist das Injektorventil zwischen der Hauptleitung und der Pumpe angeordnet. In dem letzteren Fall ist es möglich, die Pumpe in der Entnahmeleitung auch dazu zu verwenden, entsprechendes Reagenz, bei entsprechender Schaltung des Injektorventils, in den Behälter zu pumpen.

Bevorzugt ist weiterhin, dass die Injektoreinrichtung einen Vorratsbehälter zum Bevorraten von Reagenz und eine Injektorpumpe zum Pumpen von Reagenz aus dem Vorratsbehälter in die Entnahmeleitung aufweist. In diesem Fall ist das Injektorventil vorzugsweise zwischen der Pumpe der Entnahmeleitung und dem Behälter angeordnet, da zum Injizieren von Reagenz eine separate Injektorpumpe vorgesehen ist. Mittels dieser Injektorpumpe ist Reagenz aus dem Vorratsbehälter in die Entnahmeleitung und so in den Behälter injizierbar. Vorzugsweise weist die Injektorpumpe eine ausreichende Genauigkeit auf, um Reagenz in gewünschter Genauigkeit in die Entnahmeleitung zu injizieren.

In einer besonders bevorzugten Ausführungsform ist das Injektorventil als Multiportventil ausgebildet und mit einer Reagenzschleife derart verbunden, dass ein definiertes Volumen an Reagenz vor dem Injizieren in die Entnahmeleitung in diese vorlegbar ist. Bevorzugt ist beispielsweise ein Sechsportventil oder Achtportventil. Eine Reagenzschleife ist mit zwei Ports dieses Ventils verbunden und weist ein definiertes Volumen auf. Der Vorratsbehälter ist mit zwei weiteren Ports verbunden, ebenso wie die Entnahmeleitung mit zwei weiteren Ports des Multiportventils verbunden ist. Durch entsprechende Schaltung ist es nun möglich, aus dem Vorratsbehälter ein definiertes Volumen an Reagenz in die Reagenzschleife vorzulegen, anschließend das Multiportventil zu schalten, um so das definierte Volumen aus der Reagenzschleife der Entnahmeleitung zuzuführen. Dies ist bevorzugt, wenn beispielsweise eine geringe aber genaue Menge eines Reagenz zu einem Volumen an entnommenem Medium zugeführt werden soll, wie etwa eine Enzymlösung oder ein Färbemittel. Durch geeignete Schaltung ist es ebenso möglich, ein entsprechendes Volumen an zu entnehmendem Medium über die Reagenzschleife vorzulegen, so dass dieses genau abgemessen ist. Diese Schaltung ist bevorzugt, wenn beispielsweise ein kleines, genau abgemessenes Volumen aus der Hauptleitung entnommen und insbesondere automatisiert mit einer anderen Flüssigkeit verdünnt werden soll.

Die eingangs genannte Aufgabe wird ferner gelöst durch ein Verfahren zur Entnahme einer Probe eines zu entnehmenden Mediums aus einem Prozessstrom mittels einer Entnahmevorrichtung gemäß einer der vorstehend beschriebenen Ausführungsformen, umfassend die Schritte: a) Pumpen eines zu entnehmenden Mediums aus der Hauptleitung des Schlauchsystems durch die erste Entnahmeleitung in den ersten Behälter, b) Verschließen der Entnahmeleitung mittels der Pumpe, und c) Ablösen der Entnahmevorrichtung von der Hauptleitung zur Vereinzelung der Proben, wobei der erste Behälter auch nach dem Ablösen mittels der Pumpe mediendicht verschlossen ist. Vorzugsweise wird dies mittels einer Pumpe, welche in der Entnahmeleitung angeordnet ist, bewirkt. Es soll verstanden werden, dass das Verfahren zur Entnahme einer Probe eines zu entnehmenden Mediums aus einem Prozessstrom und die zuvor beschriebene Entnahmevorrichtung eine Vielzahl von gleichen bzw. ähnlichen Aspekten betreffen; insofern wird bezüglich der Vorteile und speziellen Ausgestaltung auf die obige Beschreibung vollumfänglich Bezug genommen.

Gemäß einer bevorzugten Ausführungsform des Verfahrens weist dieses ferner den folgenden Schritt auf: d) Pumpen eines zu entnehmenden Mediums aus einer Hauptleitung eines Schlauchsystems durch eine zweite Entnahmeleitung in einen zweiten Behälter. Bevorzugt wird auch dieser Schritt durch eine Pumpe bewirkt, die in der zweiten Entnahmeleitung vorgesehen ist.

Bevorzugt ist ferner der folgende Schritt vorgesehen: e) Pumpen von Spülmedium, vorzugsweise steriler Luft, von der Entnahmeleitung in die Hauptleitung. Bevorzugt wird ferner der Schritt des Verschließens der Entnahmeleitung, insbesondere mittels einer Pumpe, sowohl vor dem Pumpen eines zu entnehmenden Mediums aus einer Hauptleitung eines Schlauchsystems durch eine Entnahmeleitung in einen Behälter, als auch nach diesem Schritt ausgeführt. Dadurch ist die Entnahmeleitung außerhalb eines Pumpvorgangs mediendicht verschlossen.

Weiterhin ist bevorzugt, dass das Verfahren den Schritt: Schalten eines Selektorventils zum Verbinden der Entnahmeleitung mit einem ausgewählten Behälter von zwei oder mehr Behältern aufweist. Vorzugsweise wird dieser Schritt vor dem Schritt des Pumpens eines zu entnehmenden Mediums aus der Hauptleitung eines Schlauchsystems durch eine erste Entnahmeleitung in einen ersten Behälter ausgeführt. Nach dem Pumpen findet vorzugsweise ein weiterer Schritt des Schaltens des Selektorventils statt, zum Verbinden der Entnahmeleitung mit einem weiteren ausgewählten Behälter der zwei oder mehr Behälter. Anschließend wird vorzugsweise wiederum ein Schritt des Pumpens eines zu entnehmenden Mediums aus der Hauptleitung des Schlauchsystems durch die Entnahmeleitung in den weiteren Behälter durchgeführt. So ist es möglich, durch Schalten des Schaltventils mittels einer einzigen Pumpe und eines einzigen Verbindungspunkts zwischen Hauptleitung und Entnahmeleitung beziehungsweise eines einzigen Verbindungspunkts zwischen Hauptleitung und Entnahmeleitung und jeweils einer Pumpe in jedem Zweig der Entnahmeleitung, zu entnehmendes Medium aus der Hauptleitung in eine Mehrzahl an Behältern zu führen.

In einer bevorzugten Weiterbildung des Verfahrens umfasst dieses den Schritt: Injizieren von Reagenz in die Entnahmeleitung, vorzugsweise wenigstens teilweise während dem Schritt a) Pumpen eines zu entnehmenden Mediums aus einer Hauptleitung eines Schlauchsystems durch eine erste Entnahmeleitung in einen ersten Behälter. Dieser Schritt des Injizierens erfolgt vorzugsweise mittels einer Injektorpumpe. Dadurch ist ein Reagenz, die je nach Verfahren erforderlich sein kann, um eine entnommene Probe weiterzuverarbeiten beziehungsweise zu vermessen, gezielt in die Entnahmeleitung eingeführt.

Gemäß einer bevorzugten Weiterbildung des Verfahrens wird dabei Reagenz vor dem Injizieren in die Entnahmeleitung in eine Reagenzschleife mit einem definierten Volumen vorgelegt. Dadurch ist es möglich, Reagenz sehr genau abzumessen und zuzuführen. In der Regel weisen verwendbare Pumpen eine begrenzte Genauigkeit auf, sodass es bevorzugt sein kann, Reagenz zunächst in eine Reagenzschleife vorzulegen, um so ein genau determiniertes Volumen an Reagenz in den Behälter zuzuführen.

Gemäß einem weiteren Aspekt der Erfindung wird die eingangs genannte Aufgabe durch eine Schlauchsystem-Anordnung gelöst, umfassend: eine Hauptleitung; wenigstens zwei Entnahmevorrichtungen nach einer der vorstehend beschriebenen bevorzugten Ausführungsformen einer Entnahmevorrichtung, wobei die Entnahmeleitungen der Entnahmevorrichtungen mit der Hauptleitung verbindbar oder verbunden sind; und eine der Entnahmevorrichtung entsprechende Anzahl von Behältern zur Aufnahme von zu entnehmendem Medium, wobei jeweils ein Behälter mit einer Entnahmeleitung medienleitend verbunden ist. Für die Vorteile und speziellen Ausgestaltungen der Entnahmevorrichtung wird auf die obige Beschreibung zur bevorzugten Ausführungsform einer Entnahmevorrichtung verwiesen. Ein derartiges Schlauchsystem ist bevorzugt vormontiert und aus Kunststoff hergestellt und sterilisiert. Als Sterilisationsverfahren werden bevorzugt die bekannten Verfahren unter Verwendung von Ethylenoxid, Gammabestrahlung oder Dampfsterilisation eingesetzt.

Gemäß einem weiteren Aspekt der Erfindung wird die eingangs genannte Aufgabe ferner gelöst durch die Verwendung einer erfindungsgemäßen Entnahmevorrichtung, vorzugsweise nach einer der vorhergehenden bevorzugten Ausführungsformen einer erfindungsgemäßen Entnahmevorrichtung, oder einer erfindungsgemäßen Schlauchsystem-Anordnung, vorzugsweise nach einer vorhergehend beschriebenen bevorzugten Ausführungsform einer erfindungsgemäßen Schlauchsystem-Anordnung, zum Entnehmen einer Probe aus einem Prozessstrom eines biotechnologischen, pharmazeutischen, medizinischen oder lebensmitteltechnischen Prozesses, insbesondere Herstellungsprozesses.

Die Erfindung wird nachfolgend anhand mehrerer Ausführungsbeispiele unter Bezugnahme auf die beiliegenden Figuren näher beschrieben. Es zeigen:
- Fig. 1:: Ein bekanntes Schlauchsystem mit Schlauchverbindern und Quetschventilen;
- Fig. 2:: ein Detail aus Figur 1;
- Fig. 3:: ein herkömmliches Schlauchsystem mit Quetschventilen, bei dem die Entnahmeleitungen fest mit der Hauptleitung verbunden sind;
- Fig. 4A-4C:: jeweils einen Schritt eines Verfahrens zur Entnahme einer Probe mit dem herkömmlichen Schlauchsystem mittels Quetschventilen;
- Fig. 5:: eine erste erfindungsgemäße Entnahmevorrichtung mit einer Hauptleitung verbunden;
- Fig. 6:: eine zweite erfindungsgemäß Entnahmevorrichtung mit DCV mit einer Hauptleitung verbunden;
- Fig. 7:: eine dritte erfindungsgemäße Entnahmevorrichtung, wobei die Entnahmeleitung teilweise englumig ausgebildet ist;
- Fig. 8:: eine vierte erfindungsgemäße Entnahmevorrichtung, wobei die Entnahmeleitung mit einem Ende fest mit der Hauptleitung verbunden ist und an dem Ende ein Rückhaltemittel aufweist;
- Fig. 9:: ein Detail aus Figur 8;
- Fig. 10:: eine fünfte erfindungsgemäße Entnahmevorrichtung mit einem Schlauchverbinder;
- Fig. 11:: eine sechste erfindungsgemäße Entnahmevorrichtung mit einem Schlauchverbinder;
- Fig. 12:: eine siebte erfindungsgemäße Entnahmevorrichtung mit Hohlnadel;
- Fig. 13:: eine achte Ausführungsform einer Entnahmevorrichtung mit mehreren Behältern und einem Selektorventil;
- Fig. 14:: eine neunte Ausführungsform einer Entnahmevorrichtung mit mehreren Behältern und einem Selektorventil und einer Spüleinrichtung;
- Fig. 15:: eine zehnte Ausführungsform einer Entnahmevorrichtung mit einer Injektionseinrichtung;
- Fig. 16a:: eine Detailansicht eines als 8-Port-Ventil ausgebildeten Injektorventils in einer ersten Schaltstellung; und
- Fig. 16b:: eine Detailansicht eines als 8-Port-Ventil ausgebildeten Injektorventils in einer zweiten Schaltstellung.

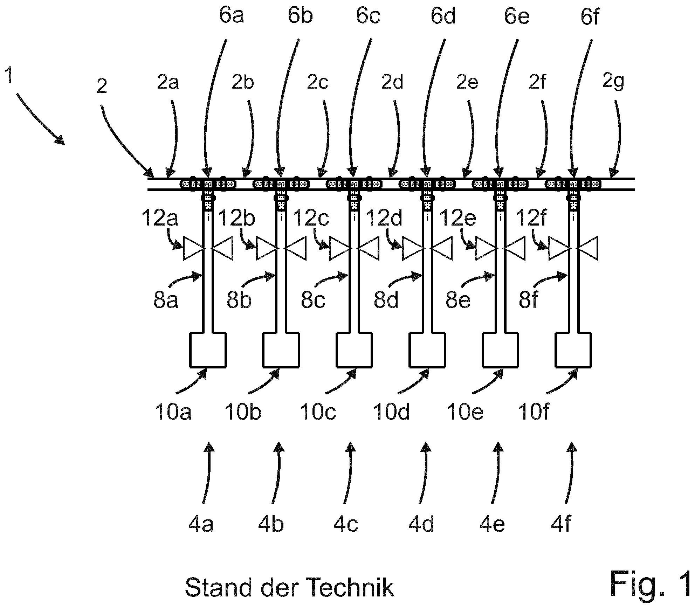

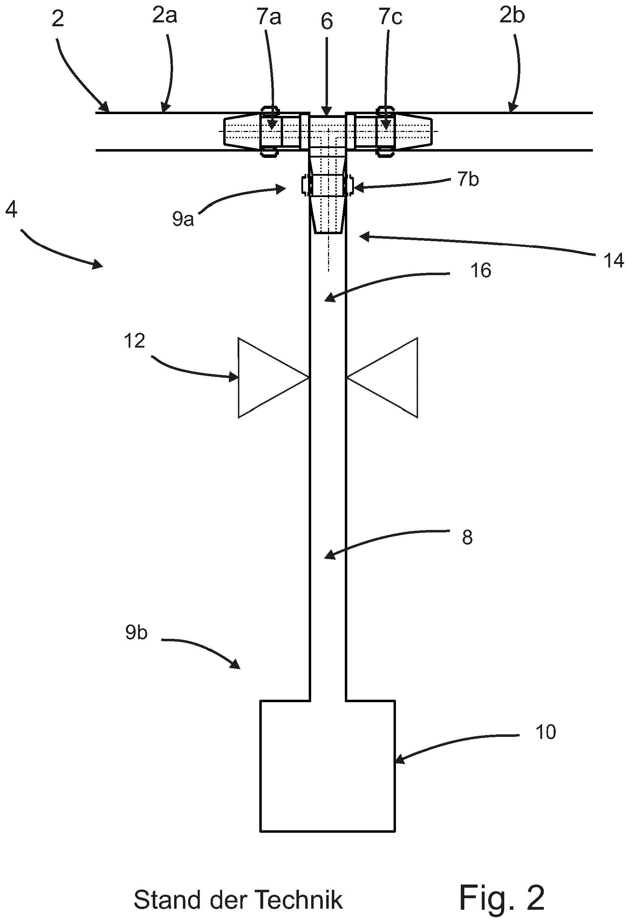

Ein herkömmliches Schlauchsystem 1 (Figur 1), auch Manifold genannt, weist eine Hauptleitung 2 sowie mehrere mit dieser verbundenen Entnahmevorrichtungen 4a-f (insgesamt mit 4 bezeichnet; siehe auch Figur 2) auf. Die Hauptleitung 2 ist gemäß Figur 1 aus mehreren kurzen Schlauchstücken 2a-g ausgebildet, die jeweils mittels eines T-Stücks 6a-f (insgesamt mit 6 bezeichnet) miteinander verbunden sind. An dem dritten T-Stückanschluss 7b (siehe Figur 2; bezogen auf Figur 1 senkrecht nach unten) ist die Entnahmeleitung 8a-f (insgesamt mit 8 bezeichnet) der Entnahmevorrichtungen 4 aufgesteckt. Die Entnahmeleitung 8 ist an ihrem anderen (bezogen auf Figur 1) unteren Ende 9b (siehe Figur 2) jeweils mit einem Behälter 10a-f (insgesamt mit 10 bezeichnet) einstückig verbunden. Ferner sind in Figur 1 Quetschventile 12a-f (insgesamt mit 12 bezeichnet) dargestellt, mittels denen die Entnahmeleitungen 8a-f einzeln absperrbar sind. Dadurch ist zu entnehmendes Medium, welches durch die Hauptleitung 2 strömt, jeweils einzeln durch die Entnahmeleitungen 8a-f in jeweils einen Behälter 10a-f führbar. Die Quetschventile 12 sind nicht Teil des Schlauchsystems, sondern eines nicht gezeigten automatischen Entnahmesystems. Die Quetschventile sind von Hand oder mittels einer elektrischen oder elektronischen Steuerung betätigbar.

In Figur 2 ist eine einzelne Entnahmevorrichtung 4 aus Figur 1, die über einen Schlauchverbinder 6 mit einer Hauptleitung 2 verbunden ist, als Detail dargestellt. Wie besonders gut aus Figur 2 ersichtlich ist, weist der Schlauchverbinder 6 drei Anschlüsse 7a, 7b, 7c auf. Auf die beiden sich gegenüberliegenden Anschlüsse 7a, 7c ist jeweils ein Schlauchstück 2a, 2b der Hauptleitung aufgesteckt. Auf den dritten freien Anschluss 7b des Schlauchverbinders 6 ist ein erstes Ende 9a der Entnahmeleitung 8 aufgesteckt. Die Entnahmeleitung 8 ist hier als flexibler Schlauch ausgeführt. Das zweite Ende 9b der Entnahmeleitung 8 ist einstückig mit einem Behälter 10 verbunden, der hier als Beutel ausgebildet ist.

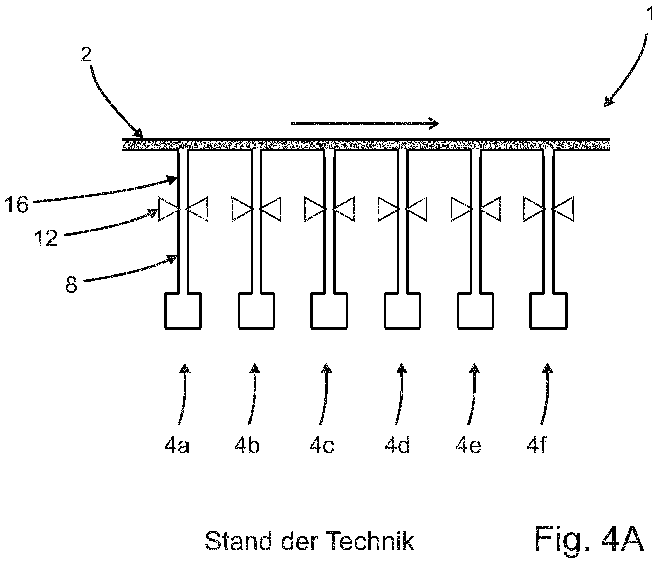

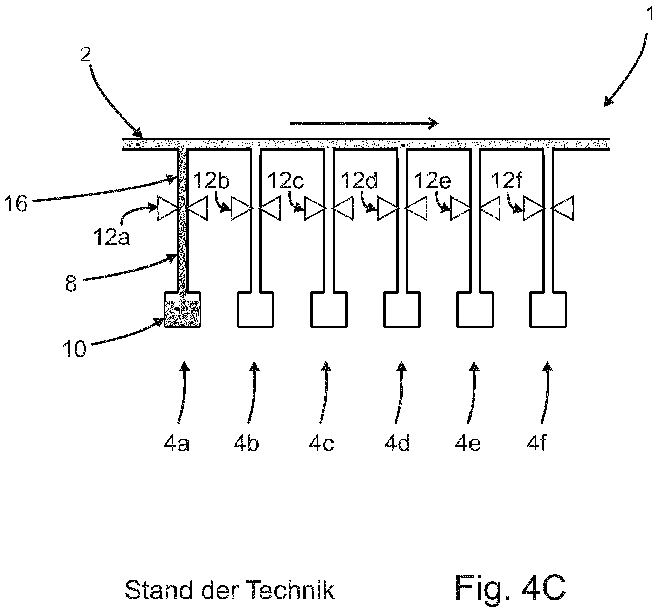

Da das Quetschventil 12 nicht unmittelbar an dem ersten Ende 9a der Entnahmeleitung angeordnet ist, bildet sich in einem ersten Abschnitt 14 der Entnahmeleitung 8 ein Totvolumen 16. Dieses Totvolumen ist definiert als Volumen zwischen dem Übergang der Hauptleitung 2 in die Entnahmeleitung 8, also von dem Schlauchverbinder 6 nämlich der Abzweigung des Anschlusses 7b bis zum Quetschventil 12. Die Wirkung des Totvolumens 16 wird mit Bezug auf die Figuren 4A-C weiter unten detaillierter beschrieben.

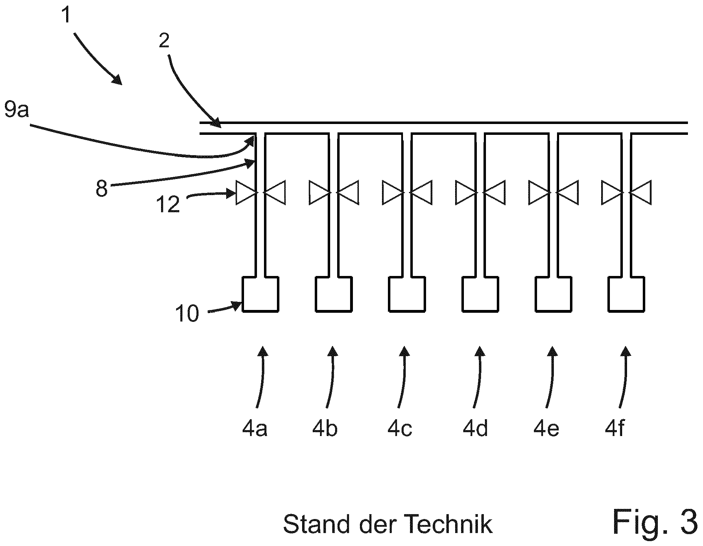

In Figur 3 ist ein weiteres bekanntes Schlauchsystem 1 dargestellt. Das Schlauchsystem 1, auch Manifold genannt, weist ebenfalls eine Hauptleitung 2 sowie mehrere Entnahmevorrichtungen 4a-f auf, die im Unterschied zu der Ausführungsform gemäß den Figuren 1 und 2 einstückig mit der Hauptleitung 2 verbunden sind. Dazu ist das erste Ende 9a der Entnahmeleitung 8 einstückig mit der Hauptleitung 2 verbunden, beispielsweise mittels Schweißen oder Kleben oder die Entnahmeleitung 8 ist einstückig an die Hauptleitung 2 angespritzt.

Das Verfahren zur Entnahme eines Mediums bei diesen bekannten Vorrichtungen ist in den Figuren 4A-C schematisch dargestellt. Die Hauptleitung 2 (siehe Figur 4A) des Schlauchsystems 1 wird gemäß dieser Darstellung (Figuren 4A-C) von links nach rechts (siehe Pfeil) mit einem Medium beströmt. Dazu kann die Hauptleitung 2 insbesondere mittels eines sterilen Verbinders mit einem Behälter eines Prozesses verbunden sein oder selbst einen Teil einer Prozessleitung bilden. Gemäß Figur 4A sind sämtliche Quetschventile 12 (in Figur 4A nur eines mit Bezugszeichen versehen) geschlossen. Die Totvolumina 16 (in Figur 4A nur eines mit Bezugszeichen versehen) sind mit Luft oder einem sterilen Gas gefüllt, so dass das in der Hauptleitung strömende Medium nicht ohne Weiteres in die Entnahmeleitung 8 eintritt.

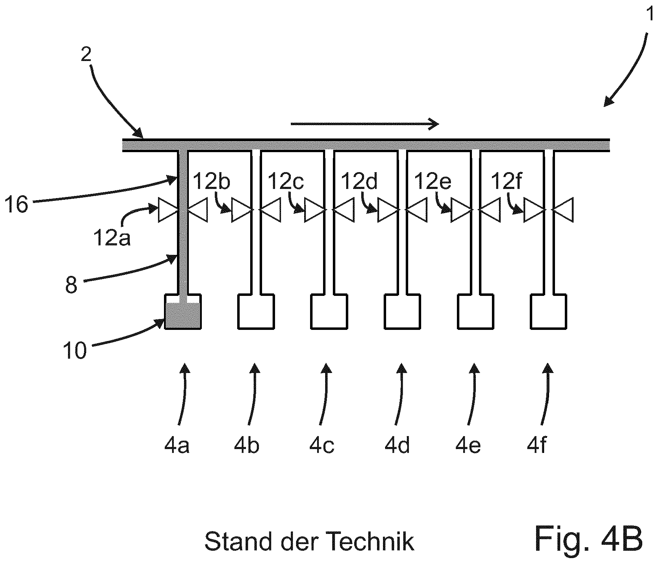

Wird nun ein erstes Quetschventil 12a geöffnet (siehe Figur 4B) strömt zu entnehmendes Medium von der Hauptleitung 2 durch die Entnahmeleitung 8 in den Behälter 10. Ist eine entsprechende Menge des zu entnehmenden Mediums in dem Behälter 10, wird das Quetschventil 12a wieder geschlossen. In dem Totvolumen 16a verbleibt jedoch ein Rest-Medium.

Wird nun in einem nachfolgenden Schritt wiederum ein Medium durch die Hauptleitung 2 geführt, beispielsweise Medium von einem nachfolgenden Prozessschritt eines biotechnologischen Prozesses oder Medium aus demselben Prozessschritt wie in Figur 4A, jedoch zu einem späteren Zeitpunkt, kann sich dieses mit dem in dem Totvolumen 16a befindlichen Medium von dem vorherigen Prozessschritt vermischen und so die nachfolgenden Probenentnahmen verunreinigen.

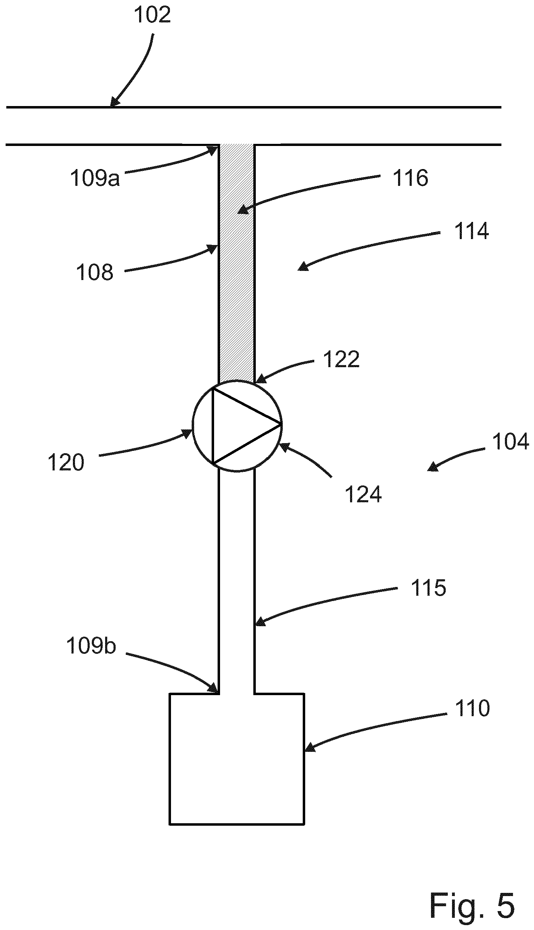

Figur 5 zeigt ein erstes Ausführungsbeispiel einer erfindungsgemäßen Entnahmevorrichtung 104 zum Gewinnen einer Probe eines zu entnehmenden Mediums aus einem Prozessstrom. Die Entnahmevorrichtung 104 umfasst eine Entnahmeleitung 108, die mit einer Hauptleitung 102 eines Schlauchsystems verbunden ist. Die Entnahmeleitung 108 ist vorgesehen zu entnehmendes Medium aus der Hauptleitung 102 in einen Behälter 110 zu leiten. Ferner weist die Entnahmevorrichtung 104 eine in der Entnahmeleitung 108 angeordnete Pumpe 120 auf, zum Pumpen von zu entnehmenden Medium aus der Hauptleitung 102 in den Behälter 110. Die Pumpe 120 weist einen Pumpeneinlass 122 und ein Pumpenauslass 124 auf. Die Entnahmeleitung 108 ist gemäß diesem Ausführungsbeispiel aus einem ersten Abschnitt 114 und einem zweiten Abschnitt 115 ausgebildet, die jeweils aus flexiblen Schlauchstücken geformt sein können. Die gesamte Entnahmeleitung 108 besteht aus dem ersten Abschnitt 114, der Pumpe 120 und dem zweiten Abschnitt 115. Die Entnahmeleitung 108 bildet eine medienleitende Verbindung zwischen der Hauptleitung 102 und dem Behälter 110. Das erste Ende 109a der Entnahmeleitung 108 ist gemäß diesem Ausführungsbeispiel einstückig mit der Entnahmeleitung 102 verbunden. Das zweite Ende 109b der Entnahmeleitung 108 ist einstückig mit dem Behälter 110 verbunden. Der erste Abschnitt 114 der Entnahmeleitung 108 weist das erste Ende 109a der Entnahmeleitung 108a auf und ist mit dem Pumpeneinlass 122 verbunden. Der zweite Abschnitt 115 der Entnahmeleitung 108 weist das zweite Ende 109b der Entnahmeleitung 108 auf und ist mit dem Pumpenauslass 124 verbunden. Dazu können an Pumpeneinlass 122 und Pumpenauslass 124 beispielsweise Schlauchverbinder angeordnet sein, auf die die Abschnitte 114, 115 der Entnahmeleitung 108 aufgesteckt werden können. Alternativ können die Abschnitte 114, 115 mittels Kleben oder Schweißen mit Pumpeneinlass 122 und Pumpenauslass 124 verbunden sein. Auch können sie bei der Herstellung direkt einstückig an dem Pumpeneinlass 122 und den Pumpenauslass 124 angespritzt werden.

Gemäß diesem ersten Ausführungsbeispiel (Figur 5) ist die Pumpe 120 in Strömungsrichtung etwa bei der der Hälfte in der Entnahmeleitung 108 angeordnet. Dadurch ist im Abschnitt 114 ein Totvolumen 116 gebildet. Das Totvolumen 116 erstreckt sich in Strömungsrichtung von dem ersten Ende 109a bis zum Pumpeneinlass 122. In radialer Richtung wird das Totvolumen 116 durch beispielsweise den Schlauch des Abschnitts 114 begrenzt.

Die Pumpe 120 weist vorzugsweise zwei Betriebszustände auf, nämlich einen Ruhezustand, in dem die Pumpe kein Medium fördert und die Entnahmeleitung 108 mediendicht verschließt, und einen Betriebszustand, in dem die Pumpe 120 Medium aus der Hauptleitung 102 in den Behälter 10 pumpt. Als Pumpen können beispielsweise Pumpen aus der Gruppe der Rotor/Stator-Systeme, Kolbenpumpen oder Verdrängerpumpen verwendet werden. Eine derartige Pumpe, die vorteilhaft bei der vorliegenden Erfindung verwendet werden kann, ist beispielsweise in WO 2007/074 363 A1 offenbart. Indem die Pumpe 120 im Ruhezustand die Entnahmeleitung 108 mediendicht verschließt, ist eine Schlauchsystem-Anordnung bzw. Manifold, in der die Entnahmevorrichtung 104 gemäß der vorliegenden Erfindung verwendet wird, stets in einem sicheren Zustand. Auch bei einem Bewegen, Verschwenken oder ähnlichem des Schlauchsystems mitsamt den Entnahmevorrichtungen 104 wird verhindert, dass Medium aus einem Behälter 110 in einen weiteren Behälter 110 gelangt und so Proben verunreinigt werden.

Zur späteren Vereinzelung der gesammelten Proben oder Aliquote in den Behältern 110 kann die jeweilige Entnahmevorrichtung 104 von der Hauptleitung 102 abgelöst werden. Beispielsweise kann der Abschnitt 114 mittels eines Messers oder dergleichen durchtrennt werden. Der Behälter 110 ist dann immer noch mittels der Pumpe 120 mediendicht verschlossen. Alternativ kann die Hauptleitung 102 zwischen den einzelnen Abzweigungen der Entnahmeleitung 108 durchtrennt oder mittels Verschweißen verschlossen und bei Bedarf gleichzeitig getrennt werden. In jedem Fall ist eine sichere Entnahme der Schlauchsystem-Anordnung bzw. Manifolds von dem System möglich, da die Pumpe 120 die jeweiligen Behälter 110 mediendicht verschließt.

Der Antrieb für die einzelnen Pumpen 120 kann beispielsweise in einer Entnahmeapparatur, die auch eine Halterung für eine Hauptleitung 102 und die Entnahmeleitungen 108 sowie die Behälter 110 bietet, fest installiert und integriert sein und beispielsweise über eine Motorwelle einzelne Rotoren oder dergleichen von Pumpen 120 antreiben. So ist eine zentrale Steuerung der Pumpen 120 beispielsweise mittels eines Steuerungssystems einer Entnahmeapparatur automatisiert möglich. Alternativ können Antriebe für Pumpen 120 individuell an entsprechenden Stellen auf die entsprechenden Entnahmevorrichtungen 104 montiert werden und die einzelne Pumpe 120 separat antreiben. Derartige Antriebe können ebenfalls zentral mittels einer Steuerung gesteuert werden; alternativ ist auch eine manuelle Steuerung möglich.

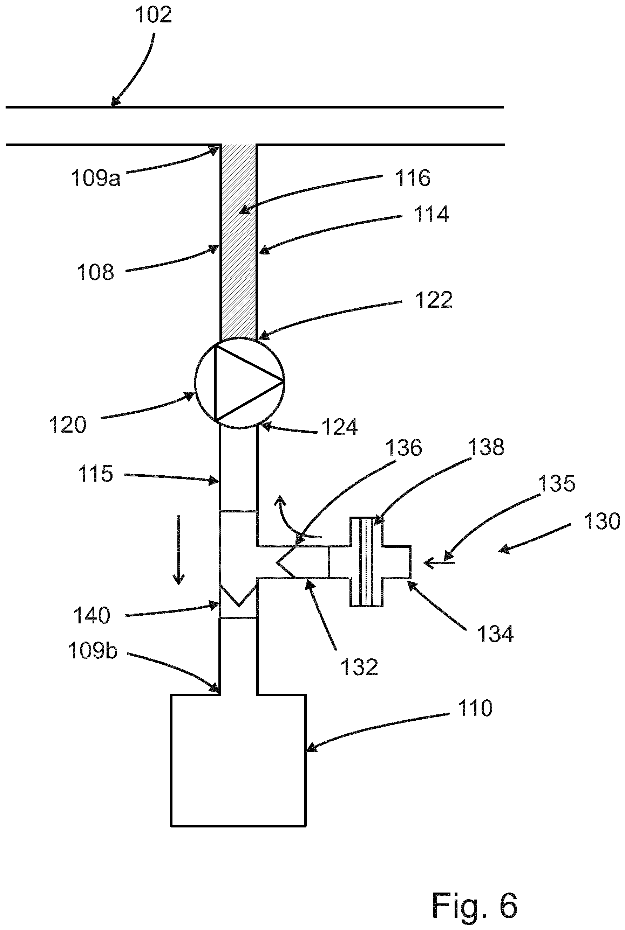

Figur 6 zeigt ein zweites Ausführungsbeispiel der Erfindung. Gleiche und ähnliche Elemente sind mit gleichen Bezugszeichen versehen. Insofern wird vollumfänglich auf die obige Beschreibung zum ersten erfindungsgemäßen Ausführungsbeispiel (Figur 5) Bezug genommen.

Grundsätzlich ist die Entnahmevorrichtung 104 gemäß dem zweiten Ausführungsbeispiel (Figur 6) identisch mit dem ersten Ausführungsbeispiel (Figur 5). Der wesentliche Unterschied liegt darin, dass gemäß diesem Ausführungsbeispiel (Figur 6) in dem Abschnitt 115 der Entnahmeleitung 108 eine Spüleinrichtung 130 angeordnet ist. Die Spüleinrichtung 130 ist dafür vorgesehen, ein Spülmedium in die Entnahmeleitung 108 einzuführen, um so die Entnahmeleitung 108 insbesondere das Totvolumen 116 sowie vorzugsweise auch die Hauptleitung 102 von Medium freizuspülen und insbesondere auch zu sterilisieren. Dazu weist die Spüleinrichtung 130 eine mit der Entnahmeleitung 108 verbundene Spülmediumzuführleitung 132 auf. Die Spülmediumzuführleitung 132 weist einen Einlass 134 auf, durch den, wie mittels des Pfeils 135 angezeigt, Spülmedium in die Spülmediumzuführleitung 132 zuführbar ist. Der Einlass 134 ist beispielsweise an eine Luftquelle, eine Quelle sterilen Gases oder einfach die Umgebung anschließbar. In der Spülmediumzuführleitung 132 ist ferner ein Rückschlagventil 136 angeordnet, welches Spülmedium von dem Einlass 134 zur Entnahmeleitung 108 strömen lässt, jedoch ein Strömen von der Entnahmeleitung 108 in Richtung des Einlasses 134 verhindert. So wird verhindert, dass zu entnehmendes Medium aus der Entnahmeleitung 108 durch die Spülmediumzuführleitung 132 aus der Entnahmevorrichtung 104 entweichen kann. In Strömungsrichtung vordem Rückschlagventil 136 ist gemäß diesem Ausführungsbeispiel (Figur 6) zusätzlich ein steriler Filter 138 vorgesehen, der die Spülmediumzuführleitung 132 durchsetzt. Der sterile Filter 138 ist dazu eingerichtet, in den Einlass 134 eintretende Umgebungsluft steril zu filtern. Indem der sterile Filter 138 in Strömungsrichtung vor dem Rückschlagventil 136 angeordnet ist, verhindert dieses zusätzlich, dass zu entnehmendes Medium mit dem sterilen Filter 138 in Kontakt kommt und diesen so kontaminiert.

In dem Abschnitt 115 der Entnahmeleitung 108 ist zusätzlich zwischen dem Pumpenauslass 124 und dem zweiten Ende 109b der Entnahmeleitung 108 ein zweites Rückschlagventil 140 vorgesehen. Das Rückschlagventil 140 ist dazu eingerichtet, einen Durchtritt von zu entnehmendem Medium von der Entnahmeleitung 102 in Richtung des Behälters 110 zuzulassen, jedoch eine Strömung von zu entnehmendem Medium von dem Behälter 110 in Richtung der Entnahmeleitung 102 zu verhindern. Hierdurch wird ermöglicht, dass das Spülmedium mittels der Pumpe 120 förderbar ist. Dazu weist die Pumpe 120 gemäß diesem Ausführungsbeispiel (Figur 6) vorzugsweise einen dritten Betriebszustand auf, in dem die Pumpe dazu eingerichtet ist, Medium vom Pumpenauslass 124 zum Pumpeneinlass 122 zu pumpen. Bei diesem zum normalen Pumpenbetrieb umgekehrten Betriebszustand ist es möglich, mittels der Pumpe 120 ein Spülmedium durch den Einlass 134 zu saugen und das Spülmedium durch das Totvolumen 116 zu drücken, sodass dieses freigespült wird. Das Rückschlagventil 140 verhindert dabei, dass bereits in dem Behälter 110 befindliches Medium mittels der Pumpe 120 wieder hinausgesaugt wird. Diese Anordnung der Spüleinrichtung 130 mit den zwei Rückschlagventilen 136, 140 wird auch als "Dual-Check-Valve" (DCV) bezeichnet.

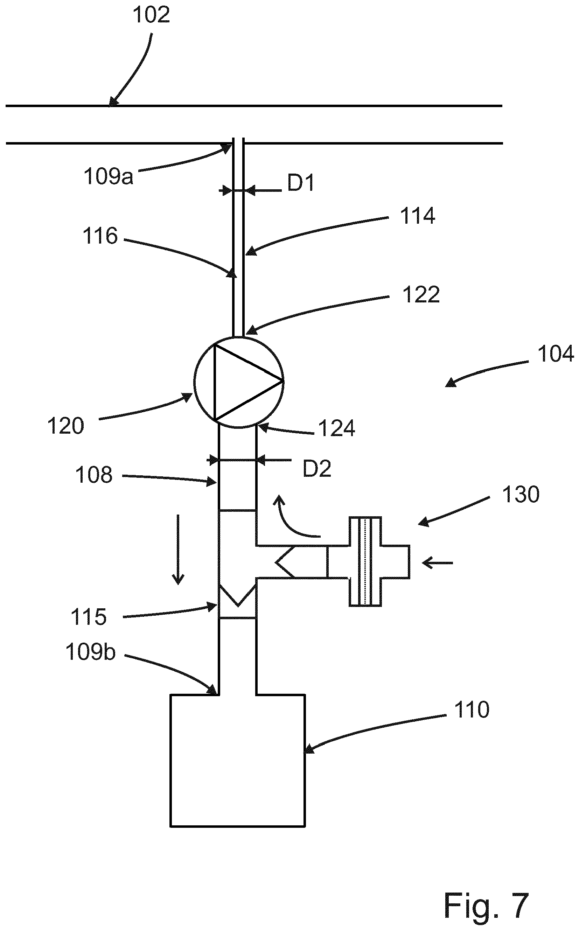

Die Entnahmevorrichtung 104 gemäß dem dritten Ausführungsbeispiel (Figur 7) ist im Wesentlichen identisch zu den ersten beiden Ausführungsbeispielen (Figur 5 und 6) gebildet. Gleiche und ähnliche Elemente sind mit gleichen Bezugszeichen versehen. Insofern wird vollumfänglich auf die obige Beschreibung zu den ersten beiden Ausführungsbeispielen Bezug genommen.

Auch die Entnahmevorrichtung 104 gemäß dem dritten Ausführungsbeispiel (Figur 7) weist eine Entnahmeleitung 108 auf, in der eine Pumpe 120 angeordnet ist. Die Entnahmeleitung 108 ist mit einem Behälter 110 verbunden. In einem Abschnitt 115 ist eine Spüleinrichtung 130 vorgesehen. Insofern deckt sich das dritte Ausführungsbeispiel mit dem zweiten Ausführungsbeispiel (Figur 6). Im Unterschied zu den ersten beiden Ausführungsbeispielen (Figur 5, 6) ist der Abschnitt 114 der Entnahmeleitung 108 englumig ausgebildet. Dies bedeutet, dass der Durchmesser D2 des ersten Abschnitts 114, der sich zwischen der Entnahmeleitung 102 von dem ersten Ende 109a bis zum Pumpeneinlass 122 erstreckt, wesentlich geringer ausgebildet ist als der Durchmesser D1 der Hauptleitung 102. Vorzugsweise ist der Durchmesser D2 gleich 0,5 D1, insbesondere 0,25, besonders bevorzugt 0,1 mal D1. Indem der Abschnitt 114 englumig ausgebildet ist, ist einerseits das sich in dem Abschnitt 114 bildende Totvolumen 116 wesentlich verkleinert, andererseits wird dadurch zu entnehmendes Medium, welches in der Entnahmeleitung 102 strömt, abgehalten, selbsttätig in die Entnahmeleitung 108 insbesondere in Abschnitt 114 zu strömen. Vorzugsweise ist der Durchmesser D2 derart bemessen, dass zu entnehmendes Medium aus der Hauptleitung 102 nur unter Betrieb der Pumpe 120 in den Abschnitt 114 strömt.

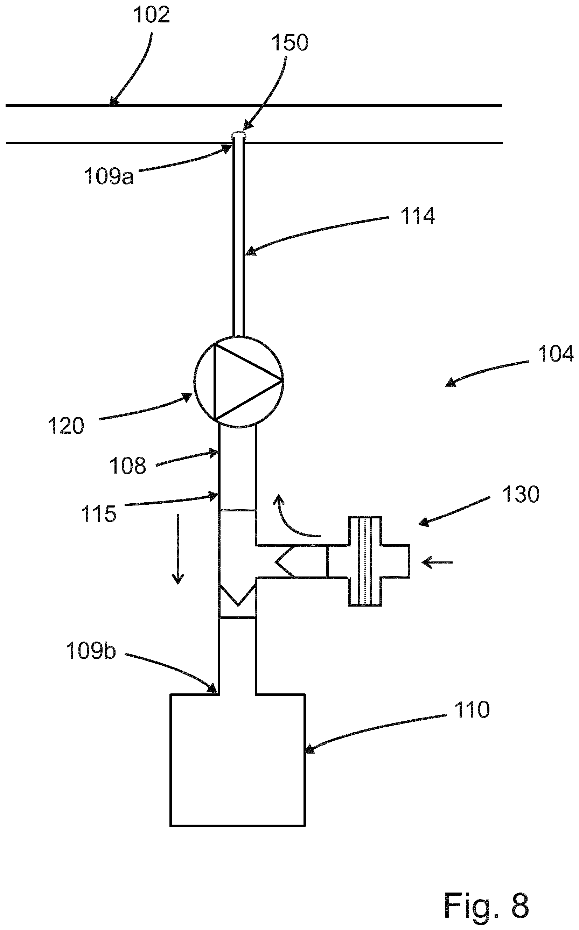

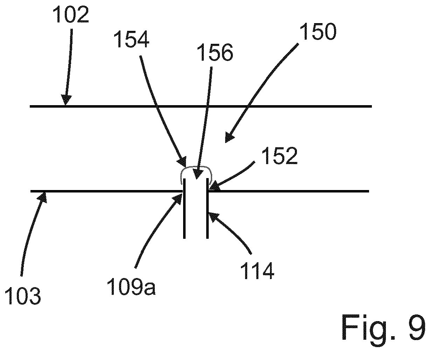

Die Figuren 8 und 9 illustrieren ein viertes Ausführungsbeispiel der Erfindung. Gleiche und ähnliche Elemente sind mit gleichen Bezugszeichen versehen. Insofern wird vollumfänglich auf die Beschreibung zu den obigen drei Ausführungsbeispielen (Figuren 5, 6 und 7) Bezug genommen.

Im Wesentlichen ist das vierte Ausführungsbeispiel (Figuren 8, 9) identisch mit dem dritten Ausführungsbeispiel (Figur 7) gestaltet. Im Unterschied zum dritten Ausführungsbeispiel (Figur 7) weist die Entnahmevorrichtung 104 gemäß dem vierten Ausführungsbeispiel (Figuren 8, 9) ein Rückhaltemittel 150 auf (siehe insbesondere Figur 9). Die Entnahmeleitung 108 ist mittels des ersten Endes 109a fest mit der Hauptleitung 102 verbunden. Der Abschnitt 114 der Entnahmeleitung 108 durchstößt eine Wandung 103 der Entnahmeleitung 102 und ragt mit einem kurzen Abschnitt 152 in die Entnahmeleitung 102 hinein. Das Rückhaltemittel 150 ist gemäß diesem Ausführungsbeispiel (Figuren 8, 9) als Kappe 154 ausgebildet, die dem ersten Ende 109a zugeordnete Öffnung 156 verschließt. Durch diese Kappe 154 wird verhindert, dass durch die Hauptleitung 102 strömendes, zu entnehmendes Medium selbsttätig in die Entnahmeleitung 108 gelangt. Die Kappe 104 ist beispielsweise aus einem dünnwandigen Silikonmaterial gebildet, welches unter Beaufschlagung eines Überdrucks, der mittels der Pumpe 120 aufgebracht wird, zerreißt und so den Einlass 156 freigibt. Dieser Überdruck ist beispielsweise bei einem Spülen mit steriler Luft mittels der Pumpe 120 aufbringbar. Alternativ kann die Pumpe 120 auch einen Unterdruck aufbringen, indem sie Medium von der Hauptleitung 120 ansaugt.

In einer Alternative ist die Kappe 154 als hydrophobe Membran ausgebildet (in den Figuren nicht gezeigt). Eine derartige Membran kann etwa aus einem Netz aus hydrophobem Material, wie etwa Polyethylen bestehen, wobei die einzelnen Durchlässe des Netzes derart gewählt sind, dass zu entnehmendes Medium bei einer vorbestimmten Druckdifferenz zwischen Hauptleitung 102 und Entnahmeleitung 108, welche mittels der Pumpe 120 aufgebracht wird, durch die Membran strömt. Eine derartige Membran anstelle einer zerreißenden Kappe hat den Vorteil, dass die Membran nach Abschluss eines Entnahmevorgangs, das heißt nach Abschluss einer Probenentnahme in einen Behälter 110, wiederum die Entnahmeleitung verschließt und so auch in nachfolgenden Entnahmeschritten zu entnehmendes Medium nicht in das Totvolumen 116 der entsprechenden Entnahmevorrichtung 104 gelangen kann.

Weiter alternativ kann das Rückhaltemittel 150 als hydrophobe Beschichtung auf einer inneren Oberfläche der Entnahmeleitung 108 ausgebildet sein oder wenigstens ein Abschnitt 114 der Entnahmeleitung 108 ist aus hydrophobem Material gebildet (in den Figuren nicht gezeigt).

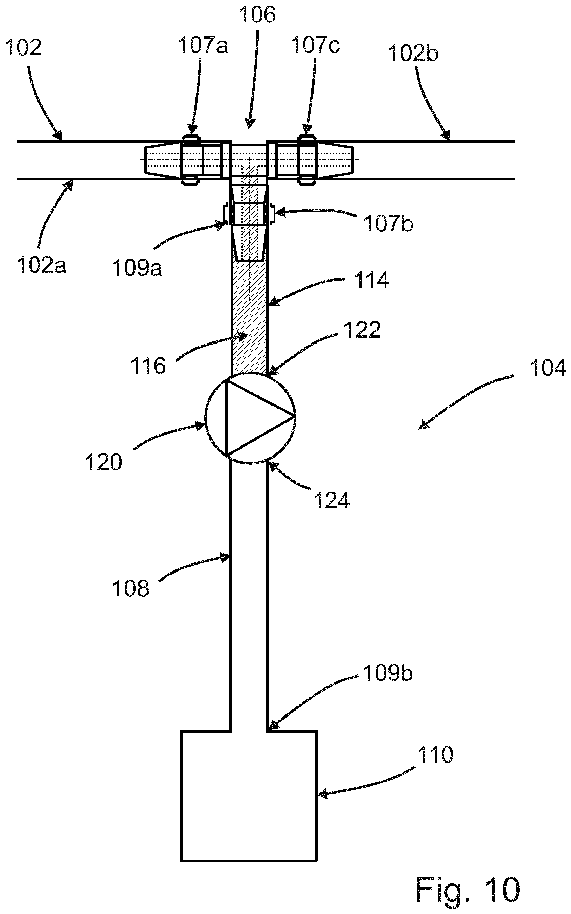

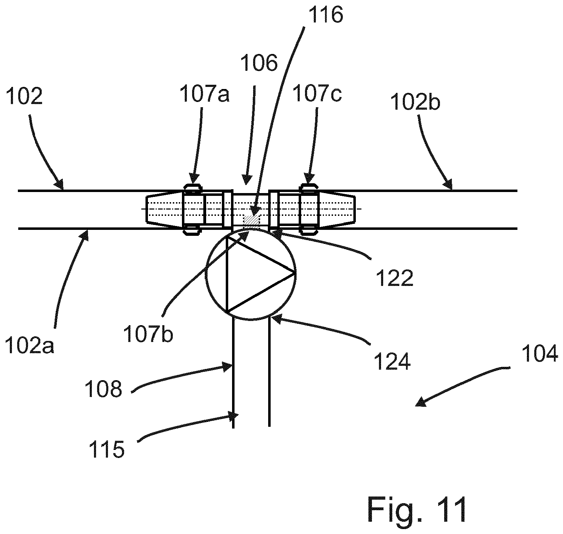

Die Figuren 10, 11 illustrieren zwei weitere Ausführungsbeispiele der Erfindung, bei der die Entnahmevorrichtung 104 über Schlauchverbinder 106 mit der Hauptleitung 102 verbunden sind. Diese zwei Ausführungsbeispiele werden nachstehend gemeinsam beschrieben. Die Ausführungsbeispiele 5 und 6 (Figuren 10, 11) sind ähnlich den vorherigen vier Ausführungsbeispielen (Figuren 5 bis 9) ausgebildet und gleiche oder ähnliche Elemente sind mit gleichen Bezugszeichen versehen. Insofern wird vollumfänglich auf die obige Beschreibung zu den ersten vier Ausführungsbeispielen Bezug genommen.

Das Ausführungsbeispiel gemäß Figur 10 ist im Wesentlichen vergleichbar mit dem ersten Ausführungsbeispiel (Figur 5) gestaltet. Im Unterschied zum ersten Ausführungsbeispiel ist bei diesem fünften Ausführungsbeispiel (Figur 10) die Entnahmevorrichtung 104 nicht einstückig mit der Hauptleitung 102 verbunden. Die Hauptleitung 102 ist aus mehreren Schlauchstücken 102a, 102b gebildet, die mittels eines Schlauchverbinders 106 miteinander verbunden sind. Der Schlauchverbinder 106 ist gemäß diesem Ausführungsbeispiel als T-Stück mit drei Anschlüssen 107a-107c ausgebildet. Zwei Anschlüsse 107a, 107c sind gegenüberliegend, ein dritter Anschluss 107b erstreckt sich senkrecht zu den beiden anderen Anschlüssen 107a, 107c. Die Schlauchstücke 102a, 102b, die einen Abschnitt der Hauptleitung 102 bilden, sind an den gegenüberliegenden Anschlüssen 107, 107c angeordnet. Die Entnahmeleitung 108 ist mittels des ersten Endes 109a an dem dritten Anschluss 107b des Schlauchverbinders 106 befestigt. Der Abschnitt 114 der Entnahmeleitung 108 erstreckt sich wiederum vom ersten Ende 109a, welches an dem Anschluss 107b des Schlauchverbinders 106 angeordnet ist, bis zum Pumpeneinlass 122. Dadurch bildet sich im ersten Abschnitt 114 ein Totvolumen 116. Vorteilhaft an dieser Ausgestaltung ist, dass nach Abschluss eines Entnahmevorgangs die Entnahmevorrichtung 104 beispielsweise manuell von dem Schlauchverbinder 106 abgezogen werden kann. Ein Durchtrennen der Entnahmeleitung 108 im Abschnitt 114 ist nicht erforderlich.

Das sechste Ausführungsbeispiel (Figur 11) zeigt eine gegenüber dem fünften Ausführungsbeispiel (Figur 10) geänderte Entnahmevorrichtung 104. Die Entnahmevorrichtung 104 gemäß diesem sechsten Ausführungsbeispiel zeichnet sich dadurch aus, dass das Totvolumen 116 minimiert ist. Die Entnahmeleitung 108 weist keinen Abschnitt 114 auf. Vielmehr ist der Anschluss 107b des Schlauchverbinders 106 kurz, im Wesentlichen als Flansch oder Anschlussstutzen, ausgebildet und direkt mit dem Pumpeinlass 122 verbunden. Besonders bevorzugt kann der Schlauchverbinder 106 einstückig mit der Pumpe 120 ausgebildet sein, beispielsweise kann der Schlauchverbinder 106 an den Einlass 122 der Pumpe 120 mittels Spritzgießen angespritzt sein.

Wie aus Figur 11 ersichtlich, ist das Totvolumen hier besonders klein. Nach Abschluss eines Entnahmevorgangs mittels der Entnahmevorrichtung gemäß dem sechsten Ausführungsbeispiel (Figur 11) kann die Hauptleitung 102 zertrennt werden, indem die einzelnen Schlauchstücke 102a, 102b von dem Schlauchverbinder 106 abgezogen werden. So sind die Entnahmevorrichtungen 104 vereinzelt und die in den Behältern 110 aufgenommenen Medien können weiter verarbeitet werden.

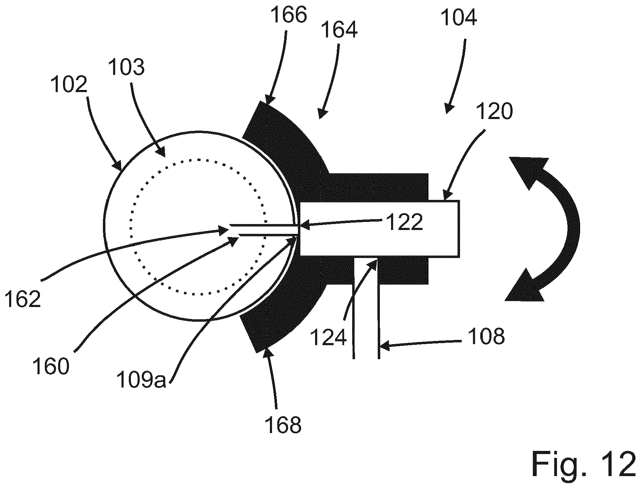

Ein siebtes erfindungsgemäßes Ausführungsbeispiel einer Entnahmevorrichtung 104 ist in Figur 12 dargestellt. Die Hauptleitung 102 ist in Figur 12 axial geschnitten dargestellt.

Gleiche oder ähnliche Elemente sind mit gleichen Bezugszeichen versehen, insofern wird vollumfänglich auf die obige Beschreibung zu den ersten sechs Ausführungsbeispielen (Figur 5 bis 11) Bezug genommen.

Die Entnahmevorrichtung 104 weist gemäß diesem Ausführungsbeispiel (Figur 12) eine Entnahmeleitung 108 auf, in der eine Pumpe 120 angeordnet ist. An einem Ende 109a der Entnahmeleitung 108 ist gemäß diesem Ausführungsbeispiel eine Hohlnadel 160 angeordnet. Die Hohlnadel 160 dient dazu, eine Wandung 103 der Hauptleitung 102 zu durchstechen. In Figur 12 ist die Wandung 103 im durchgestochenen Zustand dargestellt. Die Hohlnadel 160 erstreckt sich bis in ein Inneres der Hauptleitung 102 hinein, sodass zu entnehmendes Fluid durch einen Einlass 162 der Hohlnadel 160 in die Entnahmeleitung 108 gelangen kann. Die Hohlnadel 160 ist gemäß diesem Ausführungsbeispiel direkt an dem Pumpeneinlass 122 montiert. Dazu kann die Hohlnadel 160 beispielsweise an dem Pumpeneinlass 122 geklebt sein. In Ausführungsformen, in denen die Hohlnadel 160 aus einem Kunststoff gebildet ist, kann diese auch mit dem Pumpeneinlass 122 verschweißt sein. Auch andere Verbindungsmöglichkeiten sind denkbar. Der Pumpenauslass 124 ist gemäß dieser Ausführungsform im rechten Winkel zum Pumpeneinlass 122 angeordnet. In anderen Ausführungsbeispielen (nicht gezeigt) kann der Pumpenauslass 124 parallel, insbesondere koaxial zu dem Pumpeneinlass 122 ausgebildet sein.

Zum Abstützen der Entnahmevorrichtung 104 gegen die Hauptleitung 102 oder zum Abdichten der Durchstoßstelle von der Hohlnadel 106 in der Wandung 103 weist die Entnahmevorrichtung 104 gemäß diesem Ausführungsbeispiel ferner ein Stützelement 164 auf. Ein derartiges Stützelement 164 kann beispielsweise als Kunststoffteil ausgebildet sein, welches die Pumpe 120 wenigstens teilweise umgibt und mit dieser fest verbunden ist. Gemäß diesem Ausführungsbeispiel weist das Stützelement 164 zwei auskragende Abschnitte 166, 168 auf, die sich teilkreisförmig um die Hauptleitung 102 legen. An den beiden Abschnitten 166, 168 sind vorzugsweise Klebemittel vorgesehen, mittels denen das Stützelement 164 gegen die Hauptleitung 102 klebbar ist. Alternativ sind in den Abschnitten 166, 168 Haltemittel vorgesehen, sodass das Stützelement 164 beispielsweise mittels Kabelbindern, Schlauchschellen oder Laschen gegen die Hauptleitung 102 befestigbar ist. In einer Alternative sind die Abschnitte 166, 168 als Klipsarme ausgebildet, sodass das Stützelement 164 an der Entnahmeleitung 102 festklipsbar ist.

Eine weitere Möglichkeit der Anbindung der Entnahmevorrichtung an die Hauptleitung, die wie eingangs beschrieben auch eine Prozessanlage, wie etwa ein Behälter, Reaktor oder Leitung, sein kann, ist beispielsweise in WO 2010/008395 A1, der Fa. AllPure^{®} Technologies, Inc., New Oxford, Pennsylvania, USA, die eine Tochtergesellschaft der hiesigen Anmelderin ist, offenbart. Die darin offenbarte Vorrichtung nutzt eine Mehrzahl an hohlen Kanälen, an deren Ende eine selbstdichtende Dichtung vorgesehen ist. An die Vorrichtung sind über manuell betätigbare Schieber mehrere Entnahmeleitungen gekoppelt. Durch Betätigen der Schieber durchstößt eine Kanüle am Ende der Entnahmeleitung die selbstdichtende Dichtung und stellt so eine medienleitende Verbindung zwischen einem Beutel und dem Prozess her. Auch bei der vorliegenden Erfindung ist es denkbar, einen derartigen trommelartigen Körper zu verwenden, diesen mit einer Vielzahl an Entnahmeleitungen zu koppeln, wobei dann erfindungsgemäß in der Entnahmeleitung eine Pumpe angeordnet ist, zum Pumpen von zu entnehmendem Medium aus der Hauptleitung in den Behälter.

Eine Schlauchsystem-Anordnung, insbesondere ein Manifold, nach der vorliegenden Erfindung ist in den Figuren nicht gesondert dargestellt. Es umfasst wenigstens zwei Entnahmevorrichtungen 104, vorzugsweise nach einem der vorherigen Ausführungsbeispiele (Figuren 5 bis 12), wobei die Entnahmeleitung 108 der Entnahmevorrichtung 104 mit der Hauptleitung 102 verbindbar oder verbunden sind, und eine der Entnahmevorrichtungen 104 entsprechende Anzahl Behältern 110 zur Aufnahme von zu entnehmendem Medium, wobei jeweils ein Behälter 110 mit einer Entnahmeleitung 108 medienleitend verbunden ist. Eine derartige Schlauchsystem-Anordnung entspricht demnach im Wesentlichen einer Schlauchsystem-Anordnung gemäß den Figuren 1 oder 3, wobei die dort offenbarten Entnahmevorrichtungen 4a bis 4f durch erfindungsgemäße Entnahmevorrichtung 104 ersetzt sind. Eine derartige Schlauchsystemanordnung, insbesondere Manifold, ist vorzugsweise insgesamt vormontiert, aus Kunststoff hergestellt und sterilisiert. Es kann besonders einfach als "Single-Use-Komplettset" verwendet werden. Dadurch ist eine Gewinnung von Proben oder Aliquoten besonders einfach und sicher möglich.

Die beschriebene Schlauchsystem-Anordnung, insbesondere Manifold, und/oder die beschriebenen Entnahmevorrichtungen kann zusätzlich zu den beschriebenen Elementen geeignete invasive und/oder nicht-invasive Sensoren für Fluss, Temperatur, Druck, Trübung, Absorption, Luftblasen, Blut und ähnliches aufweisen. Dadurch ist die Funktionalität erweitert und/oder die Betriebssicherheit erhöht. Ebenfalls können weitere Elemente, wie etwa Entlüftungen, Heizelemente, Filter, Mischer, Rückschlagventile integriert werden. All diese Elemente können etwa in die Entnahmeleitung 108 und/oder die Hauptleitung 102 aufgenommen werden.

Die Schlauchsystemanordnung, insbesondere das Manifold, kann vorkonfektioniert montiert, sterilisiert und verpackt als Komplettset ausgelegt werden oder aus einzelnen Teilen zusammensetzbar sein. Es bietet sich an, etablierte Konnektoren für die Fluidleitung zu verwenden, wie beispielsweise den Luer-Verbinder (ISO 594-1:1986; deutsche Fassung EN 20594-1:1993). Vorzugsweise werden diese Verbinder aseptisch ausgelegt. Leitungen, Verbindungen und einzelne Komponenten können auch in einem Bauteil, beispielsweise einer Kassette, zusammengefasst werden, um die Handhabung für den Anwender zu erleichtern oder eine Fehlmontage zu vermeiden.

Im Inneren der Entnahmeleitung 108 und/oder der Hauptleitung 102 können zusätzlich medienberührende Sensoren angeordnet sein, wie etwa ein oder mehrere pH-Sensoren, um schon während des Prozesses Information über das zu entnehmende Medium zu gewinnen, oder bei Abfüllprozessen eine erste kontinuierliche Qualitätsbestimmung durchzuführen. Ein solcher Sensor ist vorzugsweise jede Einrichtung oder Gruppe von Einrichtungen, die in Abhängigkeit von der Anwesenheit oder Menge eines Analyten ein Messsignal erzeugt. Ein derartiger Sensor kann insbesondere ausgewählt sein aus der Gruppe bestehend aus elektrochemischen Sensoren, optischen Sensoren, amperometrischen Sensoren, Leitfähigkeitssensoren, potentiometrischen Sensoren, Biosensoren, Sauerstoffsensoren oder enzymatischen Sensoren.

Ein möglicher Verfahrensablauf zur Entnahme einer Probe eines zu analysierenden Mediums wird nachfolgend mit Bezug auf die Figuren 3, 6 und 8 beschrieben. Figur 3 zeigt zwar ein Schlauchsystem nach dem Stand der Technik, es soll jedoch verstanden werden, dass erfindungsgemäß die in Figur 3 dargestellten Entnahmevorrichtungen 4a bis 4f durch die in Figur 6 bzw. 8 dargestellte Entnahmevorrichtung 104 ersetzt sind. Figur 3 dient daher nur zu Illustrationszwecken.

Während des Prozesses, beispielsweise einem biotechnologischen, pharmazeutischen, medizinischen, oder lebensmitteltechnischen Prozess, insbesondere beim Herstellungsprozess strömt zu entnehmendes Medium durch die Hauptleitung 102. Die Hauptleitung 102 kann Teil einer Prozessleitung sein oder separat über einen Behälter oder eine eigene Pumpe gespeist werden. In diesem Ausgangszustand sind die Pumpen 120 in den Entnahmevorrichtungen 104 jeweils in einem Ruhezustand und verschließen die Entnahmeleitung 108 mediendicht. Soll nun eine erste Probe oder ein erster Aliquot gewonnen werden, wird die Pumpe 120 in einen ersten Betriebszustand versetzt. Die Pumpe 120 wird mittels des Antriebs (nicht gezeigt) derart angetrieben, dass sie Medium von der Hauptleitung 102 in den Behälter 110 fördert. Da gemäß diesem Ausführungsbeispiel (Figur 8) an der Entnahmeleitung 108 ein Rückhaltemittel 150 angeordnet ist, ist es erforderlich, dass die Pumpe 120 zunächst eine vorbestimmte Druckdifferenz zwischen der Entnahmeleitung 108 und der Hauptleitung 102 erzeugt. Ist diese Druckdifferenz erreicht, wird das Rückhaltemittel 150 durch das zu entnehmende Medium überwunden, beispielsweise reißt eine Kappe 154 oder zu entnehmendes Medium durchtritt eine hydrophobe Membran. Nun strömt zu entnehmendes Medium durch die Entnahmeleitung 108, zunächst durch den Abschnitt 114, dann durch die Pumpe 120 und schließlich durch den Abschnitt 115, durchtritt das Rückschlagventil 140 (siehe Figur 6) und gelangt in den Behälter 110. Ist die gewünschte Menge zu entnehmendes Medium in dem Behälter 110 angelangt, wird die Pumpe 120 gestoppt und verschließt die Entnahmeleitung 108 wieder mediendicht. Nun ist es bevorzugt, dass die Entnahmeleitung 108 und auch die Hauptleitung 102 gespült werden. Dazu wird die Pumpe 120 wiederum in Betrieb genommen, jedoch diesmal derart, dass Fluid von dem Pumpenauslass 124 zum Pumpeneinlass 122 (siehe Figur 6) gepumpt wird. Durch diesen Betrieb der Pumpe 120 wird Spülmedium durch den Einlass 134, die Spülmediumzuführleitung 132 und das Rückschlagventil 136 in den Abschnitt 115 gesaugt und durch den Abschnitt 114 in die Hauptleitung 102 gepumpt (siehe insbesondere Figur 6). So wird das Totvolumen 116 von restlichem zu entnehmenden Medium freigespült und bei nachfolgenden Entnahmevorgängen werden Proben nicht verunreinigt.

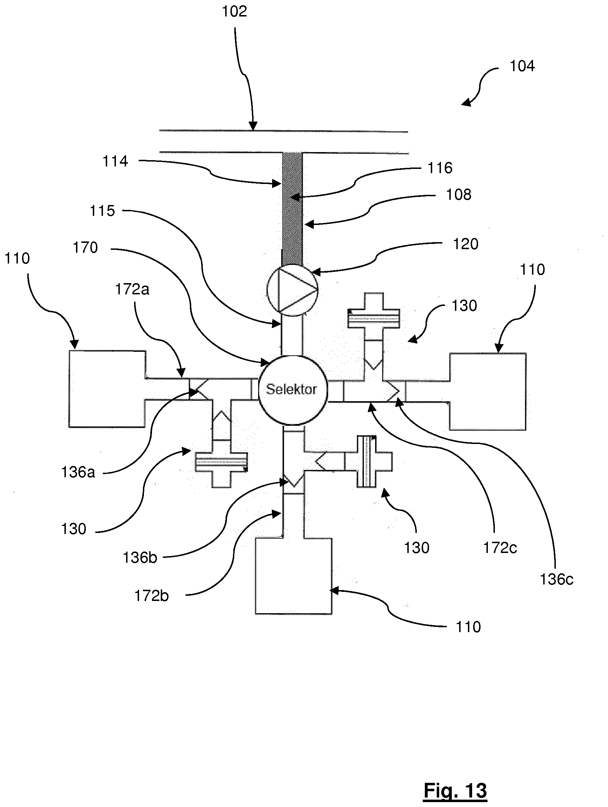

Figur 13 zeigt ein weiteres Ausführungsbeispiel der Entnahmevorrichtung 104. Die Entnahmevorrichtung 104 weist gemäß diesem Ausführungsbeispiel ein Selektorventil 170 auf, welches einlassseitig mit dem zweiten Abschnitt 115 der Entnahmeleitung 108 verbunden ist. Das Selektorventil 170 hat gemäß diesem Ausführungsbeispiel einen Einlass (verbunden mit dem zweiten Abschnitt 115) sowie drei Auslässe, die jeweils mit einem Zweig 172a, 172b, 172c der Entnahmeleitung 108 verbunden sind. Durch das Selektorventil 170 sind mit einer Entnahmeleitung 108 mehrere Behälter 110 (gemäß diesem Ausführungsbeispiel drei Stück) mit einem zu entnehmenden Medium befüllbar. Gemäß diesem Ausführungsbeispiel ist ferner jedem Zweig 172a, 172b, 172c der Entnahmeleitung 108 eine separate Spüleinrichtung 130 zugeordnet, welche im Wesentlichen wie in Figur 6 bereits beschrieben ausgebildet ist. Insofern wird vollumfänglich auf die obige Beschreibung zur Figur 6 Bezug genommen, in der gleiche und ähnliche Elemente mit den gleichen Bezugszeichen versehen sind.

Die Pumpe 120 ist in der Entnahmeleitung 108 zwischen der Hauptleitung und dem Selektorventil 170 vorgesehen. Bevorzugt ist die Pumpe 120 gemäß diesem Ausführungsbeispiel ebenso wie die Pumpe 120 in Bezug auf die Figuren 5 bis 12 beschrieben ausgebildet. Die Pumpe 120 ist in die Entnahmeleitung 108 integriert und als Einwegpumpe ausgebildet, d.h. sie kann nach einem abgeschlossenen Entnahmevorgang entsorgt werden. Die Pumpe 120 bildet somit einen Teil des Strömungskanals der Entnahmeleitung 108. Die Pumpe 120 ist, wie bereits mit Bezug auf die Figuren 5 und 6 beschrieben, mit einen Einlass 122 (siehe Figur 5) mit einem ersten Abschnitt 114 der Entnahmeleitung 108 verbunden und mit einem Auslass 124 (siehe Figur 5) mit dem zweiten Abschnitt 115 der Entnahmeleitung verbunden. So ist bei entsprechender Betätigung des Selektorventils 170 mit einer Pumpe 120 in drei Behälter 110 zu entnehmendes Medium pumpbar. Das Ventil kann eine automatische Verteilung des Mediums auf die verschiedenen Behälter 110 ermöglichen. Das Selektorventil 170 kann manuell oder elektrisch betätigt werden. Vorzugsweise ist eine Steuerung für das Selektorventil vorgesehen, welche das Selektorventil entsprechend einer vorgegebenen Steuerung, beispielsweise zeitabhängig, steuert.

In einer Alternative ist gemäß diesem Beispiel, das nicht Teil der Erfindung ist, vorgesehen, dass die Pumpe 120 nicht in der Entnahmeleitung angeordnet ist, sondern dass die Entnahmeleitung 108 zwischen Hauptleitung 102 und Selektorventil 170 durchgehend verläuft. In einem solchen Ausführungsbeispiel weist die Entnahmeleitung 108 nicht den ersten und zweiten Abschnitt 114, 115 auf, sondern eine einzelne durchgängige Leitung. Diese Leitung kann zum Entnehmen von Medium aus der Hauptleitung 102 mit einer peristaltischen Pumpe zusammenwirken, indem die Entnahmeleitung 108, insbesondere der Abschnitt zwischen Hauptleitung 102 und Selektorventil 170, in die peristaltische Pumpe eingelegt wird und so Medium aus der Hauptleitung 102 in die Behälter 110 gefördert wird. Entsprechende Rückschlagventile 136a, 136b, 136c in den Zweigen 172a, 172b, 172c führen dazu, dass Medium nicht aus dem Behälter 110 zurück in die Entnahmeleitung 108 oder die Hauptleitung 102 fließen kann.

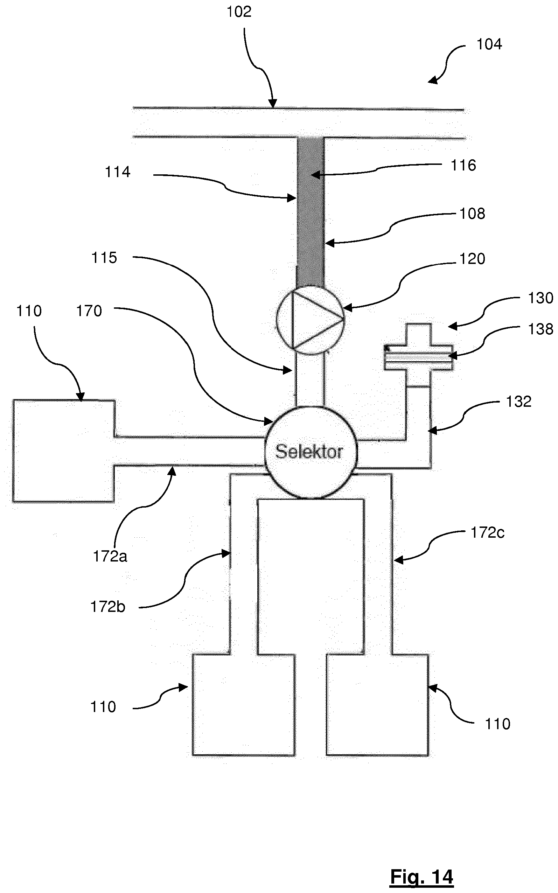

In Figur 14 ist eine weitere Variante der Entnahmevorrichtung 104 mit einem Selektorventil 170 und einer Spüleinrichtung 130 dargestellt. Im Unterscheid zu der in Figur 13 gezeigten Entnahmevorrichtung 104 ist nicht jedem Behälter 110 eine Spüleinrichtung 130 zugeordnet, sonder gemäß diesem Ausführungsbeispiel (Figur 14) ist eine einzige Spüleinrichtung 130 vorgesehen, die mittels der Spülmediumzuführleitung 132 an einen Anschluss des Selektorventils angeschlossen ist. Das Selektorventil 170 weist gemäß dieser Ausführungsform vier weitere Anschlüsse auf, wobei an einem Anschluss der Abschnitt 115 der Entnahmeleitung angeschlossen ist und an den weiteren drei die Zweige 172a, 172b und 172c der Entnahmeleitung angeschlossen sind. Wird das Selektorventil 170 nun entsprechend geschaltet, und die Pumpe 120 so betrieben, dass sie in Richtung der Hauptleitung 102 fördert, kann Spülmedium über den sterilen Filter 138, die Spülmediumzuführleitung 132, das Selektorventil 170, den Abschnitt 115, die Pumpe 120 und den Abschnitt 114 zu der Hauptleitung 102 gepumpt werden, sodass das Totvolumen 116 gespült wird. Dieser Spülvorgang kann unabhängig davon vorgenommen werden, welcher der Behälter 110 mit einer Probe befüllt werden soll.

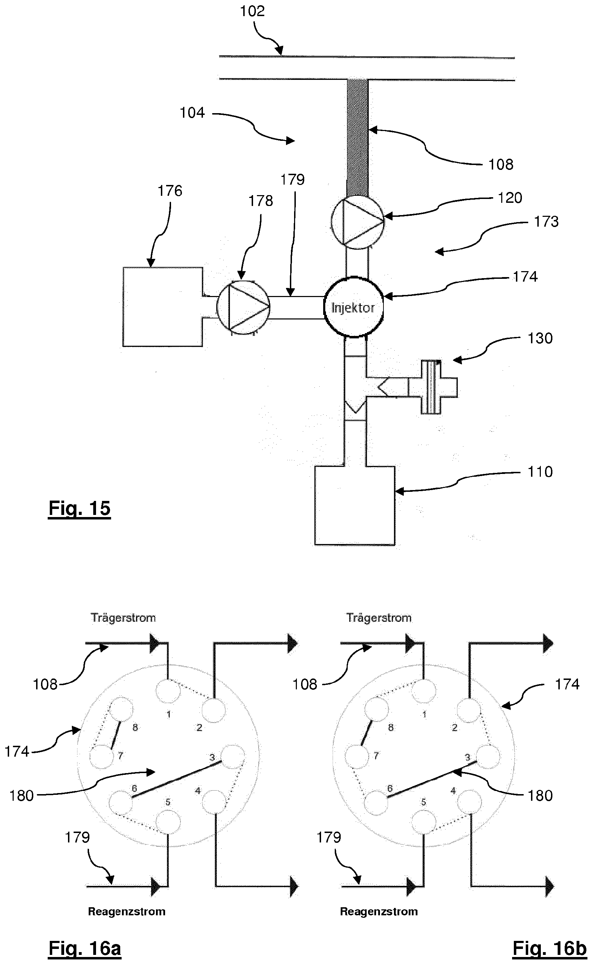

Eine weitere Ausführungsform der erfindungsgemäßen Entnahmevorrichtung 104 ist in Figur 15 dargestellt. Bei dieser Entnahmevorrichtung 104 gemäß Figur 15 ist wiederum nur ein Behälter 110 vorgesehen. Im Wesentlichen entspricht die Entnahmevorrichtung 104 gemäß Figur 15 der Entnahmevorrichtung gemäß Figur 6, und insofern wird vollumfänglich auf die obige Beschreibung zu Figur 6 Bezug genommen.

Im Unterschied zu der Entnahmevorrichtung 104 gemäß Figur 6 weist die Entnahmevorrichtung 104 gemäß Figur 15 eine Injektoreinrichtung 173 auf. Die Injektoreinrichtung 173 dient dazu, Reagenz in die Entnahmeleitung 108 zu injizieren. Die Injektoreinrichtung 173 weist ein Injektorventil 174 auf, welches in der Entnahmeleitung 108 angeordnet ist. Gemäß diesem Ausführungsbeispiel ist das Injektorventil 174 zwischen der Pumpe 120 und dem Behälter 110 angeordnet. Es ist ebenso denkbar, das Injektorventil 174 zwischen der Entnahmeleitung 102 und der Pumpe 120 anzuordnen.

Das Injektorventil weist zwei Schaltstellungen auf, eine, in der die Hauptleitung 102 mit dem Behälter 110 verbunden ist und eine zweite Stellung, in der der Vorratsbehälter 176 mit dem Behälter 110 verbunden ist. Bevorzugt ist der Vorratsbehälter 176 nicht unmittelbar mit dem Behälter 110 verbunden, sondern es ist eine Reagenzschleife 180 zwischengeschaltet, die mit Bezug auf die Figuren 16a und 16b unten genauer beschrieben werden wird. Der Vorratsbehälter 176 dient dazu, Reagenz, welche dem Behälter 110 zugeführt werden soll, zu bevorraten. Zum Injizieren weist die Injektoreinrichtung 173 ferner eine Injektorpumpe 178 auf, mittels welcher Reagenz aus dem Vorratsbehälter 176 über das Injektorventil 174 in den Behälter 110 pumpbar ist. Zwischen dem Injektorventil 174 und dem Behälter 110 ist ferner eine Spüleinrichtung 130 vorgesehen, welche identisch zu der Spüleinrichtung 130 gemäß Figur 6 ausgebildet ist. Mittels dieser Spüleinrichtung 130 ist sterile Luft sowohl mittels der Pumpe 120 als auch mittels der Pumpe 178 einsaugbar, sodass die Entnahmeleitung 108, und/oder die Leitung 179, welche das Injektorventil 174 mit dem Vorratsbehälter 176 verbindet, rückspülbar sind/ist. Hierdurch ist es möglich, die gesamte Entnahmeleitung sowie das Injektorventil und die Leitung 179 von Medien- und Reagenzresten zu befreien.

Auch bei dieser Ausführungsform ist es denkbar, dass die Pumpe 120 nicht in der Entnahmeleitung 108 angeordnet ist, wie dies bei den vorherigen Ausführungsbeispielen stets der Fall ist. Es ist ebenso denkbar, dass die Pumpe 120 als peristaltische Pumpe ausgebildet ist und die Entnahmeleitung 108 zwischen der Hauptleitung 102 und dem Injektorventil 174 einstückig ist und zum Entnehmen von Medium in eine entsprechende Ausnehmung einer peristaltischen Pumpe einlegbar ist. In einem solchen Fall ist es bevorzugt, vor dem vollständigen Entnehmen entsprechende Klemmen oder sonstige separate Verschlussmittel an den Leitungen vorzusehen, um ein Rückströmen von Medium in die Hauptleitung 102 zu verhindern. Zu einem gewissen Grad, vorzugsweise vollständig, wird dies auch bereits durch die Rückschlagventile der Spüleinrichtung 130 erreicht

Die weiteren Figuren 16a und 16b illustrieren die Funktion des Injektorventils 174 gemäß einer bevorzugten Ausführungsform. Das Injektorventil 174 ist gemäß diesem Ausführungsbeispiel als Achtportventil ausgebildet. Die Schaltung des Achtportventils ist in den Figuren 16a und 16b dargestellt. Zur Erhöhung der Genauigkeit der Zuführung von Reagenz aus dem Vorratsbehälter 176 in dem Behälter 110 ist an den Ports 3 und 6 (siehe Figuren 16a, 16b) eine Reagenzschleife 180 angeschlossen. An weiteren Ports ist einerseits die Entnahmeleitung 108 (Ports 1 und 2) sowie die Leitung 179 (Ports 4 und 5) angeschlossen. In der ersten Schaltstellung (in Figur 16a dargestellt) ist über die Schaltung (siehe gestrichelte Linien) des Injektorventils 174 die Reagenzschleife 180 mit der Leitung 179 verbunden, und Reagenz strömt durch die Reagenzschleife 180. Entsprechend ist Port 1 mit Port 2 verbunden, und zu entnehmendes Medium kann durch die Entnahmeleitung 108 strömen. Bei einer entsprechenden Umschaltung hin zu der in Figur 16b gezeigten Stellung des Injektorventils 174 wird die Entnahmeleitung 108 mit der Reagenzschleife 180 gekoppelt, sodass nun in der Reagenzschleife 180 befindliches Reagenz durch die Entnahmeleitung 108 in den Behälter 110 strömt. Dadurch ist es möglich, ein genau definiertes Volumen Reagenz von dem Vorratsbehälter 176 durch entsprechende Schaltung des Injektorventils 174 in den Behälter 110 zu fördern.

Es soll verstanden werden, dass die Ausführungsbeispiele gemäß den Figuren 13, 14 und 15 kombiniert werden können und sollen. Es ist bevorzugt, dass die Entnahmevorrichtung 104 sowohl ein Selektorventil 170 als auch eine Injektoreinrichtung 173 aufweist.

## Patentansprüche

1. Entnahmevorrichtung (104) zum Gewinnen einer Probe eines zu entnehmenden Mediums aus einem Prozessstrom, umfassend:
eine Entnahmeleitung (108), die mit einer Hauptleitung (102) eines Schlauchsystems (100) verbindbar oder lösbar verbunden ist, zum Leiten von zu entnehmendem Medium aus der Hauptleitung (102) in einen Behälter (110); und
eine in der Entnahmeleitung (108) angeordnete Pumpe (120), zum Pumpen von zu entnehmendem Medium aus der Hauptleitung (102) in den Behälter (110), wobei die Pumpe (120) im Ruhezustand einen mediendichten Verschluss der Entnahmeleitung (108) bildet,
wobei die Entnahmeleitung (108) aus einem ersten und einem zweiten Abschnitt (114, 115) besteht, wobei der erste Abschnitt (114) die Hauptleitung (102) medienleitend mit einem Pumpeneinlass (122) verbindet und der zweite Abschnitt (115) einen Pumpenauslass (124) medienleitend mit dem Behälter (110) verbindet, sodass ein Strömungspfad von der Hauptleitung (102) in den Behälter (110) durch die Pumpe (120) verläuft.

2. Entnahmevorrichtung nach Anspruch 1,
**dadurch gekennzeichnet, dass** das Schlauchsystem (100) ein Manifold-Schlauchsystem zur Entnahme und/oder Aufbewahrung von Proben und/oder Aliquoten aus einem biotechnologischen, pharmazeutischen, medizinischen oder lebensmitteltechnischen Prozess, insbesondere Herstellungsprozess ist.

3. Entnahmevorrichtung nach einem der vorherigen Ansprüche,
**dadurch gekennzeichnet, dass** die Pumpe (120) einen Pumpeneinlass (122) und einen Pumpenauslass (124) aufweist, wobei der Pumpeneinlass (122) englumig ausgebildet ist und relativ zu dem Pumpenauslass (124), der Entnahmeleitung (108) und/oder der Hauptleitung (102) einen kleineren Durchmesser aufweist.

4. Entnahmevorrichtung nach einem der vorherigen Ansprüche,
ferner umfassend eine Spüleinrichtung (130), vorzugsweise eine mit der Entnahmeleitung (108) verbundene Spülmediumzuführleitung (132), zum Zuführen von Spülmedium in die Entnahmeleitung (108).

5. Entnahmevorrichtung nach einem der vorherigen Ansprüche,
**dadurch gekennzeichnet, dass** die Entnahmeleitung (108) mit einem Ende (109a) fest mit der Hauptleitung (102) verbunden ist und an dem Ende ein Rückhaltemittel (150) aufweist.

6. Entnahmevorrichtung nach einem der vorstehenden Ansprüche,
**dadurch gekennzeichnet, dass** an einem Ende (109a) der Entnahmeleitung (108) eine Hohlnadel (160) zum Durchstechen einer Wandung (103) einer Hauptleitung (102) angeordnet ist.

7. Entnahmevorrichtung nach einem der vorstehenden Ansprüche,
**gekennzeichnet durch** einen Schlauchverbinder (106), insbesondere ein T-Stück, welcher an dem Ende (109a) der Entnahmeleitung (108) angeordnet ist.

8. Entnahmevorrichtung nach einem der vorstehenden Ansprüche,
**gekennzeichnet durch** ein Selektorventil (170), welches einlassseitig mit der Hauptleitung (102) und auslassseitig mit zwei oder mehr Behältern (110) verbunden ist, wobei das Selektorventil schaltbar ist, zum wahlweisen Verbinden der Entnahmeleitung mit einem der zwei oder mehr Behälter (110).

9. Entnahmevorrichtung nach Anspruch 4 und 8,
**dadurch gekennzeichnet, dass** die Spüleinrichtung (130) mit dem Selektorventil (170) verbunden ist.

10. Entnahmevorrichtung nach Anspruch 4 und 8,
**dadurch gekennzeichnet, dass** die Spülmediumzuführleitung (132) der Spüleinrichtung (130), zum Zuführen von Spülmedium in die Entnahmeleitung (108), zwischen dem Selektorventil und wenigstens einem Behälter (110) mit der Entnahmeleitung (108) verbunden ist.

11. Entnahmevorrichtung nach einem der vorstehenden Ansprüche,
**gekennzeichnet durch** eine Injektoreinrichtung (173) zum Injizieren eines Reagenz in die Entnahmeleitung (108).

12. Entnahmevorrichtung nach Anspruch 11,
**dadurch gekennzeichnet, dass** die Injektoreinrichtung (173) ein Injektorventil (174), einen Vorratsbehälter (176) zum Bevorraten von Reagenz und eine Injektorpumpe (178) zum Pumpen von Reagenz aus dem Vorratsbehälter (176) in die Entnahmeleitung (108) aufweist.

13. Entnahmevorrichtung nach Anspruch 12,
**dadurch gekennzeichnet, dass** das Injektorventil (174) als Multiportventil ausgebildet ist und mit einer Reagenzschleife (180) derart verbunden ist, dass ein definiertes Volumen an Reagenz vor dem Injizieren in die Entnahmeleitung (108) in diese vorlegbar ist.

14. Verfahren zur Entnahme einer Probe eines zu entnehmenden Mediums aus einem Prozessstrom mittels einer Entnahmevorrichtung (104) nach einem der vorstehenden Ansprüche 1 bis 13, das Verfahren umfassend folgende Schritte:
- Pumpen eines zu entnehmenden Mediums aus der Hauptleitung (102) des Schlauchsystems durch die erste Entnahmeleitung (108) in den ersten Behälter (110);
- Verschließen der Entnahmeleitung (108) mittels der Pumpe (120);
- Ablösen der Entnahmevorrichtung (104) von der Hauptleitung (102) zur Vereinzelung der Proben, wobei der erste Behälter (110) auch nach dem Ablösen mittels der Pumpe (120) mediendicht verschlossen ist.

15. Verfahren nach Anspruch 14, ferner umfassend den Schritt:
- Schalten eines Selektorventils zum Verbinden der Entnahmeleitung (108) mit einem ausgewählten Behälter (110) von zwei oder mehr Behältern (110).

16. Verfahren nach Anspruch 14 oder 15, ferner umfassend den Schritt:
- Injizieren von Reagenz in die Entnahmeleitung (108) mittels einer Injektoreinrichtung (173), vorzugsweise wenigstens teilweise während dem Schritt: Pumpen eines zu entnehmenden Mediums aus einer Hauptleitung (102) eines Schlauchsystems durch eine erste Entnahmeleitung (108) in einen ersten Behälter (110).

17. Verfahren nach Anspruch 16, wobei Reagenz vordem Injizieren in die Entnahmeleitung (108) in eine Reagenzschleife (180) mit einem definierten Volumen vorgelegt wird.

18. Schlauchsystem-Anordnung, umfassend
eine Hauptleitung (102);
wenigstens zwei Entnahmevorrichtungen (104) nach einem der Ansprüche 1 bis 13, wobei die Entnahmeleitungen (108) der Entnahmevorrichtungen (104) mit der Hauptleitung (102) verbindbar oder verbunden sind; und
einer den Entnahmevorrichtungen (104) entsprechenden Anzahl Behältern (110) zur Aufnahme von zu entnehmendem Medium, wobei jeweils ein Behälter (110) mit einer Entnahmeleitung (108) medienleitend verbunden ist.

19. Verwendung einer Entnahmevorrichtung (104) nach einem der Ansprüche 1 bis 13 oder einer Schlauchsystem-Anordnung nach Anspruch 18, zum Entnehmen einer Probe aus einem Prozessstrom eines biotechnologischen, pharmazeutischen, medizinischen oder lebensmitteltechnischen Prozesseses, insbesondere Herstellungsprozesses.

20. Entnahmevorrichtung nach einem der Ansprüche 1 bis 13 oder Schlauchsystem-Anordnung nach Anspruch 18,
**dadurch gekennzeichnet, dass** es als
a) Single-Use-Komplettset ausgebildet ist;
b) sterilisiert ist; und/oder
c) vorkonfektioniert ist.

## Claims

1. An extraction device (104) for obtaining a sample of a medium to be extracted from a process stream, comprising:
an extraction line (108), which can be connected or is detachably connected to a main line (102) of a hose system (100), for conducting medium to be extracted from the main line (102) to a container (110); and
a pump (120) arranged in the extraction line (108) for pumping medium to be extracted from the main line (102) to the container (110), wherein the pump (120) forms a media tight closure of the extraction line (108) in the resting state,
wherein the extraction line (108) consists of a first and a second section (114, 115), wherein the first section (114) connects the main line (102) in a media conducting manner to a pump inlet (122) and the second section (115) connects a pump outlet (124) in a media conducting manner to the container (110), so that a flow path runs through the pump (120) from the main line (102) to the container (110).

2. The extraction device according to claim 1,
**characterized in that** the hose system (100) is a manifold hose system for extraction and/or storage of samples and/or aliquots from a biotechnological, pharmaceutical, medicinal or food technology process, in particular a manufacturing process.

3. The extraction device according to any of the foregoing claims,
**characterized in that** the pump (120) has a pump inlet (122) and a pump outlet (124), wherein the pump inlet (122) has a narrow bore and has a smaller diameter relative to the pump outlet (124), the extraction line (108) and/or the main line (102) .

4. The extraction device according to any of the foregoing claims,
further comprising a purging device (130), preferably a purging medium feed line (132) connected to the extraction line (108), for feeding purging medium to the extraction line (108).

5. The extraction device according to any of the foregoing claims,
**characterized in that** the extraction line (108) is rigidly connected with one end (109a) to the main line (102) and has a retaining means (150) on the end.

6. The extraction device according to any of the foregoing claims,
**characterized in that** a hollow needle (160) for piercing a wall (103) of a main line (102) is arranged on one end (109a) of the extraction line (108).

7. The extraction device according to any of the foregoing claims,
**characterized by** a hose connector (106), in particular a tee, which is arranged on the end (109a) of the extraction line (108) .

8. The extraction device according to any of the foregoing claims,
**characterized by** a selector valve (170) which is connected on the inlet side to the main line (102) and on the outlet side to two or more containers (110), wherein the selector valve is switchable, for optional connecting the extraction line to one of the two or more containers (110).

9. The extraction device according to claim 4 and 8,
**characterized in that** the purging device (130) is connected to the selector valve (170).

10. The extraction device according to claim 4 and 8,
**characterized in that** the purging medium feed line (132) of the purging device (130) is connected to the extraction line (108) for feeding purging medium to the extraction line (108), between the selector valve and at least one container (110).

11. The extraction device according to any of the foregoing claims,
**characterized by** an injector device (173) for injecting a reagent into the extraction line (108).

12. The extraction device according to claim 11,
**characterized in that** the injector device (173) has an injector valve (174), a reservoir container (176) for storing reagent and an injector pump (178) for pumping reagent from the reservoir container (176) into the extraction line (108).

13. The extraction device according to claim 12,
**characterized in that** the injector valve (174) is embodied as a multiport valve and is connected to a reagent loop (180) such that a defined volume of reagent can be placed in it prior to injection into the extraction line (108).

14. A method for extracting a sample of a medium to be extracted from a process stream by means of an extraction device (104) according to any of the foregoing claims 1 to 13, said method comprising the following steps of:
- pumping a medium to be extracted from the main line (102) of the hose system through the first extraction line (108) into the first container (110);
- sealing the extraction line (108) by means of the pump (120) ;
- removing the extraction device (104) from the main line (102) for isolating the samples, wherein the first container (110) is sealed media-tight also after removal by means of the pump (120).

15. The method according to claim 14, further comprising the step of:
- switching a selector valve for connecting the extraction line (108) to a selected container (110) from two or more containers (110).

16. The method according to claim 14 or 15, further comprising the step of:
- injecting reagent into the extraction line (108) by means of an injector device (173), preferably at least partially during the step of: pumping a medium to be extracted from the main line (102) of the hose system through the first extraction line (108) into the first container (110).

17. The method according to claim 16, wherein reagent is placed into a reagent loop (180) with a defined volume prior to injection into the extraction line (108).

18. A hose system arrangement, comprising
a main line (102);
at least two extraction devices (104) according to any of claims 1 to 13, wherein the extraction lines (108) of the extraction devices (104) can be connected or are connected to the main line (102); and
a number of containers (110) corresponding to the extraction devices (104) for receiving medium to be extracted, wherein in each case a container (110) is connected in a media conducting manner to an extraction line (108).

19. The use of an extraction device (104) according to any of claims 1 to 13 or of a hose system arrangement according to claim 18 for extracting a sample from a process stream of a biotechnological, pharmaceutical, medicinal or food technology process, in particular a manufacturing process.

20. An extraction device according to any of claims 1 to 13 or a hose system arrangement according to claim 18, **characterized in that** it is
a) embodied as a single-use complete set;
b) sterilized; and/or
c) pre-finished.

## Revendications

1. Dispositif de prélèvement (104) destiné à obtenir un échantillon d'un milieu à prélever depuis un courant de processus, comprenant :
une conduite de prélèvement (108) qui peut être reliée ou est reliée de façon amovible à une conduite principale (102) d'un système de tubes (100), pour conduire un milieu à prélever depuis la conduite principale (102) jusque dans un récipient (110) ; et
une pompe (120) agencée dans la conduite de prélèvement (108), pour pomper un milieu à prélever depuis la conduite principale (102) jusque dans le récipient (110), dans lequel la pompe (120) forme, à l'état de repos, une fermeture étanche au milieu de la conduite de prélèvement (108),
dans lequel la conduite de prélèvement (108) est constituée d'une première et d'une seconde section (114, 115), dans lequel la première section (114) relie, de façon à conduire un milieu, la conduite principale (102) à une admission de pompe (122), et la seconde section (115) relie, de façon à conduire un milieu, une évacuation de pompe (124) au récipient (110), de sorte qu'un chemin d'écoulement se déroule à travers la pompe (120) depuis la conduite principale (102) jusque dans le récipient (110).

2. Dispositif de prélèvement selon la revendication 1, **caractérisé en ce que** le système de tubes (100) est un système de tubes collecteur pour le prélèvement et/ou la conservation d'échantillons et/ou d'aliquotes depuis un processus biotechnologique, pharmaceutique, médical ou de technologie alimentaire, en particulier un processus de fabrication.

3. Dispositif de prélèvement selon l'une des revendications précédentes,
**caractérisé en ce que** la pompe (120) présente une admission de pompe (122) et une évacuation de pompe (124), dans lequel l'admission de pompe (122) est conçue avec un lumen étroit et présente un diamètre inférieur par rapport à l'évacuation de pompe (124), à la conduite de prélèvement (108) et/ou à la conduite principale (102).

4. Dispositif de prélèvement selon l'une des revendications précédentes,
comprenant en outre un équipement de rinçage (130), de préférence une conduite d'amenée de milieu de rinçage (132) reliée à la conduite de prélèvement (108), pour amener un milieu de rinçage jusque dans la conduite de prélèvement (108).

5. Dispositif de prélèvement selon l'une des revendications précédentes,
**caractérisé en ce que** la conduite de prélèvement (108) est solidement reliée à la conduite principale (102) avec une extrémité (109a) et présente à l'extrémité un moyen de retenue (150).

6. Dispositif de prélèvement selon l'une des revendications précédentes,
**caractérisé en ce qu'**à une extrémité (109a) de la conduite de prélèvement (108) est agencée une aiguille creuse (160) pour transpercer une paroi (103) d'une conduite principale (102).

7. Dispositif de prélèvement selon l'une des revendications précédentes,
**caractérisé par** un raccord de tube (106), en particulier un té, lequel est agencé à l'extrémité (109a) de la conduite de prélèvement (108).

8. Dispositif de prélèvement selon l'une des revendications précédentes,
**caractérisé par** une valve sélecteur (170), laquelle est reliée à la conduite principale (102) sur le côté admission et à deux ou plusieurs récipients (110) sur le côté évacuation, dans lequel la valve sélecteur peut être commutée pour relier la conduite de prélèvement au choix à l'un des deux ou plusieurs récipients (110).

9. Dispositif de prélèvement selon la revendication 4 et 8,
**caractérisé en ce que** l'équipement de rinçage (130) est relié à la valve sélecteur (170).

10. Dispositif de prélèvement selon la revendication 4 et 8,
**caractérisé en ce que** la conduite d'amenée de milieu de rinçage (132) de l'équipement de rinçage (130) est reliée à la conduite de prélèvement (108), entre la valve sélecteur et au moins un récipient (110), pour amener un milieu de rinçage jusque dans la conduite de prélèvement (108).

11. Dispositif de prélèvement selon l'une des revendications précédentes,
**caractérisé par** un équipement injecteur (173) pour injecter un réactif dans la conduite de prélèvement (108).

12. Dispositif de prélèvement selon la revendication 11,
**caractérisé en ce que** l'équipement injecteur (173) comprend une valve d'injecteur (174), un récipient de stockage (176) pour stocker un réactif et une pompe d'injection (178) pour pomper du réactif depuis le récipient de stockage (176) jusque dans la conduite de prélèvement (108).

13. Dispositif de prélèvement selon la revendication 12,
**caractérisé en ce que** la valve d'injecteur (174) est conçue en tant que valve multiport et est reliée à une boucle de réactif (180) de telle sorte qu'un volume défini de réactif puisse être présenté au préalable dans celle-ci avant l'injection dans la conduite de prélèvement (108).

14. Procédé destiné à prélever un échantillon d'un milieu à prélever depuis un courant de processus au moyen d'un dispositif de prélèvement (104) selon l'une des revendications précédentes 1 à 13, le procédé comprenant les étapes suivantes :
- pomper un milieu à prélever depuis la conduite principale (102) du système de tubes par la première conduite de prélèvement (108) jusque dans le récipient (110) ;
- fermer la conduite de prélèvement (108) au moyen de la pompe (120) ;
- détacher le dispositif de prélèvement (104) depuis la conduite principale (102) pour individualiser les échantillons, dans lequel le premier récipient (110) est fermé de façon étanche au milieu également après le détachement au moyen de la pompe (120).

15. Procédé selon la revendication 14, comprenant en outre l'étape suivante :
- commuter une valve sélecteur pour relier la conduite de prélèvement (108) à un récipient (110) sélectionné par deux ou plusieurs récipients (110).

16. Procédé selon la revendication 14 ou 15, comprenant en outre l'étape suivante :
- injecter du réactif dans la conduite de prélèvement (108) au moyen d'un équipement injecteur (173), de préférence au moins partiellement pendant l'étape : pomper un milieu à prélever depuis une conduite principale (102) d'un système de tubes par une première conduite de prélèvement (108) jusque dans un premier récipient (110).

17. Procédé selon la revendication 16, dans lequel un réactif est présenté au préalable dans une boucle de réactif (180) avec un volume défini avant d'être injecté dans la conduite de prélèvement (108).

18. Agencement de système de tubes, comprenant
une conduite principale (102) ;
au moins deux dispositifs de prélèvement (104) selon l'une des revendications 1 à 13, dans lequel les conduites de prélèvement (108) des dispositifs de prélèvement (104) peuvent être ou sont reliées à la conduite principale (102) ; et
un nombre de récipients (110) correspondant aux dispositifs de prélèvement (104) pour la réception d'un milieu à prélever, dans lequel respectivement un récipient (110) est relié, de façon à conduire un milieu, à une conduite de prélèvement (108).

19. Utilisation d'un dispositif de prélèvement (104) selon l'une des revendications 1 à 13 ou d'un agencement de système de tubes selon la revendication 18, pour le prélèvement d'un échantillon depuis un courant de processus d'un processus biotechnologique, pharmaceutique, médical ou de technologie alimentaire, en particulier d'un processus de fabrication.

20. Dispositif de prélèvement selon l'une des revendications 1 à 13 ou agencement de système de tubes selon la revendication 18,
**caractérisé en ce qu'**il est
a) conçu en tant que jeu complet à usage unique ;
b) stérilisé ; et/ou
c) pré-confectionné.
